# EUROPÄISCHE PATENTANMELDUNG

(11) **EP 2 005 941 A2**
(43) Veröffentlichungstag der Anmeldung: **24.12.2008**
(21) Anmeldenummer: 08009757.9
(22) Anmeldetag: 29.05.2008
(51) Int. Cl.: A61K 8/33, A61K 8/34, A61K 8/37, A61K 8/97, A61Q 19/08

(54) **Zellverjüngende Zusammensetzungen**

(30) Priorität: 01.06.2007 DE 102007025874; 26.05.2008 DE 102008025089
(71) Anmelder: Henkel AG & Co. KGaA, 40589 Düsseldorf (DE)
(72) Erfinder: Holtkötter, Olaf, 50354 Hürth (DE); Knieps-Massong, Waltraud, 41352 Korschenbroich (DE); Elias, Kerstin, 50825 Köln (DE); Janßen, Frank, 41470 Neuss (DE); Jassoy, Claudia, 40237 Düsseldorf (DE); Kolbe, Anke, 41539 Dormagen (DE); Engels, Ursula, 40223 Düsseldorf (DE); Waldmann-Laue, Marianne, 40789 Monheim (DE); Träger, Anemone, 40227 Düsseldorf (DE); Heinen, Soraya, 50858 Köln (DE)

(57) **Zusammenfassung**

Die Erfindung betrifft die Verwendung von Hydroxystilbenen und/oder Hydroxystilben-Oligomeren, deren Alkylethern und/oder deren Estern, zur verjüngenden Behandlung von Zellen, insbesondere zur verjüngenden Behandlung von Zellen der Epidermis und/oder der Dermis.

## Beschreibung

Die Erfindung betrifft die Verwendung von Hydroxystilbenen und/oder Hydroxystilben-Oligomeren, deren Alkylethern und/oder deren Estern, zur verjüngenden Behandlung von Zellen, insbesondere zur verjüngenden Behandlung von Zellen der Epidermis und/oder der Dermis.

Ein wichtiges Ziel der Anti-Ageing-Kosmetik ist die Verminderung der Hautalterung und im besten Falle sogar die Verjüngung der Haut. Insbesondere der letzte Punkt ist eine besondere Herausforderung, da die verjüngende Eigenschaft einer Zusammensetzung an bestimmte zelluläre Parameter geknüpft werden muss.

Ein typisches Problem der gealterten Haut ist der fortschreitende Übergang der Hautzellen aus einem proliferativen Status in einen ruhenden, seneszenten Status. Dieser Übergang entspricht der Alterung auf zellulärer Ebene, auf den sich ein Großteil der makroskopischen Alterungseffekte zurückführen lässt. Die Zellzahl im Gewebe verringert sich und kann mangels proliferierender Zellen nicht mehr aufgefüllt werden. Dadurch können keine Reparaturen des umliegenden Gewebes durchgeführt werden, Defekte reichern sich an, wodurch die Haut atrophisch wird und erschlafft.

### Stand der Technik

Mittel und Behandlungsmethoden zur Behandlung reifer oder intrinsisch oder extrinsisch gealterter oder lichtgeschädigter Haut, insbesondere zur Antifaltenbehandlung, sind eine bedeutende kosmetische Herausforderung. Bei diesen Mitteln besteht ein Bedarf nach Inhaltsstoffen, die den Zubereitungen weiteren Nutzen verleihen, sei es aus anwendungstechnischer Sicht, aus herstelltechnischer Sicht oder aus Sicht des Verbrauchers, der beispielsweise aufgrund bestimmter Inhaltsstoffe ein verbessertes subjektives Empfinden dem kosmetischen Mittel gegenüber entwickelt. Der Schwerpunkt der Leistung eines Inhaltsstoffes kann dabei je nach kosmetischem Mittel auch unterschiedlich sein. So kann ein und derselbe Stoff in einem Shampoo oder einer Spülung pflegende Eigenschaften für die behandelten Haare bewirken, während er in einem Haarfärbemittel darüber hinaus die Anfärbung der Kopfhaut verringert oder die zum Teil aggressive Wirkung des Färbemittels kompensiert.

Die Altershaut wird kosmetisch in erster Linie mit Vitamin A-Derivaten oder Hydroxysäuren behandelt, die über eine Stimulation der Proliferation der Basalzellen in der Epidermis zu einer Verdickung der Epidermis und damit Glättung der Haut führen.

Retinoide greifen in den Metabolismus der Hautzellen ein und bewirken neben der Anregung der Proliferation und Differenzierung der epidermalen Keratinozyten die Steigerung der Collagenproduktion durch Fibroblasten. Darüber hinaus soll Retinol die Bildung von Collagen verdauenden Enzymen reduzieren (New Scientist 2031, 42-46, 1996). Retinsäure besitzt jedoch teratogene Eigenschaften und darf nur in verschreibungspflichtigen Pharmaka eingesetzt werden. Der Einsatz von Retinol in kosmetischen und pharmazeutischen topischen Praeparaten ist aus mehreren Gründen als problematisch zu betrachten. So weist Retinol eine relative hohe Phototoxizität auf und kann deshalb nur in geringen Konzentrationen in Zusammensetzungen zur Anwendung am Menschen eingesetzt werden. Darüber hinaus wird Retinol unter Einwirkung von Wärme und/oder Licht leicht oxidativ abgebaut und ist in kosmetischen und pharmazeutischen Formulierungen schwierig zu stabilisieren.

Hydroxysäuren sind in den für die Behandlung der Hautalterung notwendigen Konzentrationen häufig schlecht verträglich, insbesondere für die Behandlung empfindlicher Haut und für die Behandlung der Haut im Augenbereich.

Neuere Ansätze bestehen darin, die Proteine, die in der trockenen oder der Altershaut fehlen oder vermindert vorkommen, gezielt zu substituieren bzw. indirekt in die Stoffwechselprozesse einzugreifen, die bei trockener Haut oder mit zunehmendem Alter gestört sind, um diese zu normalisieren. Als Beispiel sei hier die Stimulation der Collagensynthese mit dem Zweck der Faltenreduzierung angeführt. Weiterhin werden beispielsweise Laminin, Substanzen zur Verlängerung der Lebenszeit von Hautzellen und bestimmte Extrakte zur Stimulierung der epidermalen Differenzierung eingesetzt. Hierbei handelt es sich teilweise jedoch um pharmakologisch wirksame Substanzen mit hohem Nebenwirkungspotenzial.

Topische und orale Zusammensetzungen, die Resveratrololigomere oder deren Ester als Antioxidans, Radikalfänger oder als gefäßschützenden Wirkstoff enthalten, waren bekannt aus FR 2766176 A1. FR 2816843 A1 offenbart Resveratrololigomere als Inhibitor des Enzyms 5-alpha-Reduktase. Weiterhin sind die Resveratrololigomere Viniferine im Stand der Technik als antibakteriell, antifungiell, anti-tumoral und als leberschützend beschrieben.

### Aufgabenstellung

Um eine progressive Hautalterung zu verhindern, müssen die Zellen der Haut reaktiviert werden und in einen jungen Status zurückgeführt werden. Der Anteil seneszenter Zellen muss verringert werden. Dadurch kann die zelluläre Ausstattung der Haut korrigiert werden und die Haut insgesamt einen jüngeren Status wiedererlangen.

Eine Aufgabe der vorliegenden Erfindung war es, gut verträgliche Wirkstoffe und Zusammensetzungen, die diese gut verträglichen Wirkstoffe enthalten, zur Verfügung zu stellen, um gealterte Zellen, insbesondere Zellen der Epidermis und/oder der Dermis, in einen jüngeren Status zurückzuführen.

Eine weitere Aufgabe der vorliegenden Erfindung war es, gut verträgliche Wirkstoffe und Zusammensetzungen, die diese gut verträglichen Wirkstoffe enthalten, zur Verfügung zu stellen, um den Übergang der Zellen der Epidermis und/oder der' Dermis aus dem proliferativen Status in den ruhenden, seneszenten Status zu verzögern.

Eine weitere Aufgabe der vorliegenden Erfindung war es, gut verträgliche Wirkstoffe und Zusammensetzungen, die diese gut verträglichen Wirkstoffe enthalten, zur Verfügung zu stellen, um den Anteil der Zellen, insbesondere der Zellen in der Epidermis und/oder der Dermis, die sich im ruhenden, seneszenten Status befinden, zu verringern.

Eine weitere Aufgabe der vorliegenden Erfindung war es, gut verträgliche Wirkstoffe und Zusammensetzungen, die diese gut verträglichen Wirkstoffe enthalten, zur Verfügung zu stellen, um der Haut einen jüngeren Phänotyp zu verleihen.

Überraschend wurde gefunden, dass kosmetische oder dermatologische Zusammensetzungen, die in einem geeigneten kosmetischen oder dermatologischen Träger mindestens eine Verbindung, ausgewählt aus den Hydroxystilben-Oligomeren, deren Alkylethern und/oder deren Estern, enthalten, die gestellten Aufgaben in hervorragender Weise lösen.

Gegenstand der vorliegenden Erfindung ist die nicht-therapeutische, kosmetische Verwendung von mindestens einer Verbindung, ausgewählt aus den Hydroxystilben-Oligomeren, deren Alkylethern und/oder deren Estern
- zur Verjüngung und/oder Revitalisierung der Zellen, insbesondere der Zellen der Epidermis und/oder der Dermis, oder
- zur Verzögerung des Übergangs der Zellen, insbesondere der Zellen der Epidermis und/oder der Dermis, aus dem proliferativen Status in den ruhenden, seneszenten Status, oder
- zur Verringerung des Anteils der Zellen, insbesondere der Zellen in der Epidermis und/oder der Dermis, die sich im ruhenden, seneszenten Status befinden, oder
- um der Haut einen jüngeren Phänotyp zu verleihen.

Gemäß einer bevorzugten Ausführungsform der Erfindung ist die mindestens eine erfindungsgemäß verwendete Verbindung, ausgewählt aus den Hydroxystilben-Oligomeren, deren Alkylethern und/oder deren Estern, in einer zur Verabreichung auf topischem oder oralem Wege und/oder durch Injektion geeigneten Zusammensetzung konfektioniert.

Ein weiterer Gegenstand der vorliegenden Erfindung ist die Verwendung von mindestens einer Verbindung, ausgewählt aus den Hydroxystilben-Oligomeren, deren Alkylethern und/oder deren Estern, zur Herstellung einer Zusammensetzung
- zur Verjüngung und /oder Revitalisierung der Zellen, insbesondere der Zellen der Epidermis und/oder der Dermis, oder
- zur Verzögerung des Übergangs der Zellen, insbesondere der Zellen der Epidermis und/oder der Dermis, aus dem proliferativen Status in den ruhenden, seneszenten Status, oder
- zur Verringerung des Anteils der Zellen, insbesondere der Zellen in der Epidermis und/oder der Dermis, die sich im ruhenden, seneszenten Status befinden, oder
- um der Haut einen jüngeren Phänotyp zu verleihen.

Gemäß einer bevorzugten Ausführungsform der Erfindung ist die mindestens eine erfindungsgemäß verwendete Verbindung, ausgewählt aus den Hydroxystilben-Oligomeren, deren Alkylethern und/oder deren Estern, in einer Form konfektioniert, die für eine Verabreichung auf topischem Wege oder oralem Wege und/oder durch Injektion geeignet ist.

Erfindungsgemäß besonders bevorzugt wird die mindestens eine Verbindung, ausgewählt aus den Hydroxystilben-Oligomeren, deren Alkylethern und/oder deren Estern, in einer Gesamtmenge von 0,00001 - 10 Gew.-%, bevorzugt 0,0001 - 1 - 5 Gew.-%, besonders bevorzugt 0,001 - 0,5 - 4 Gew.-%, außerordentlich bevorzugt 0,005 - 0,1 - 1 Gew.-%, jeweils bezogen auf das Gesamtgewicht der erfindungsgemäß verwendeten Zusammensetzung, verwendet.

Bei Hydroxystilbenen handelt es sich um eine umfangreiche Gruppe von Naturstoffen, meist Phytoalexine; viele davon werden aus Koniferen gewonnen und liegen häufig als Glycoside und/oder Methylether vor. Sie können aber auch synthetisch hergestellt werden. Auch mit Säuren, beispielsweise mit Phosphorsäure, veresterte Hydroxystilbene sind für die erfindungsgemäßen Verwendungen geeignet. Die meisten Hydroxystilbene liegen in der (E)-Form vor, einige kommen aber auch in der (Z)-Form vor. Zu den wichtigsten Vertretern zählt Pinosylvin (3,5-Stilbendiol). In der (E)-Form bildet Pinosylvin Nadeln, die in Wasser unlöslich, in Aceton löslich sind. (E)- u. (Z)-Isomere kommen neben den entsprechenden Mono- und Dimethylethern in Pinien, Kiefern und anderen Nadelhölzern vor. Pinosylvin besitzt fungizide und antibakterielle Eigenschaften. Pinosylvin-monomethylether wirkt als Fraßhemmstoff.

Resveratrol (3,4',5-Stilbentriol, 3,4',5-Trihydroxystilben, Struktur siehe allgemeine Formel RESV-I, mit R¹ = OH und R² = H) ist ein Phytoalexin aus den Wurzeln von Veratrum grandiflorum und der Rinde von Pinus sibirica und kommt unter anderem in Eucalyptus-, Polygonum- u. Nothofagus-Arten vor, insbesondere in dem Rhizom von Polygonum cuspidatum. Piceatannol (3,3',4,5'-Stilbentetrol, 3,3',4,5'-Tetrahydroxystilben, Astringenin) bildet in der (E)-Form Nadeln, wurde aus Fichten isoliert und ist ebenfalls fungitoxisch, hemmt das Pflanzenwachstum und wirkt ichthyotoxisch.

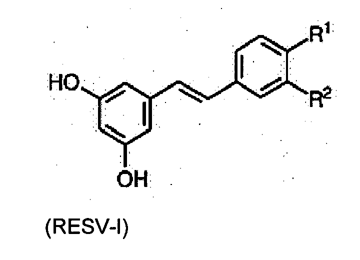

Über den Angriff einer Hydroxylgruppe an die ethylenische Doppelbindung eines weiteren Hydroxystilben-Moleküls unter Ausbildung eines Dihydrofuranrings können Hydroxystilbene oligomerisieren. Bekannte Beispiele für die Oligomere von Hydroxystilbenen sind die Dimere des Resveratrols, insbesondere des (E)-Resveratrols, sind die sogenannten Viniferine, insbesondere das trans-epsilon-Viniferin (siehe Formel RESV-II).

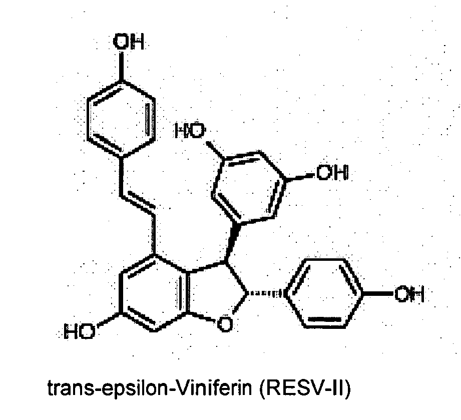

Bekannte Trimere und Tetramere des Resveratrols sind Vitisin E (siehe Formel RESV-III) und Vitisin D (siehe Formel RESV-IV). 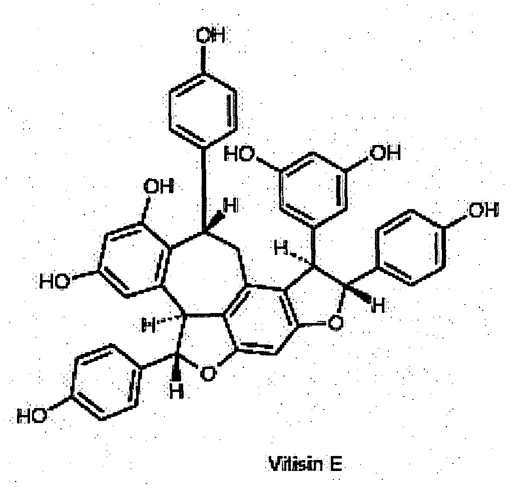 Vitisin E (RESV-III) 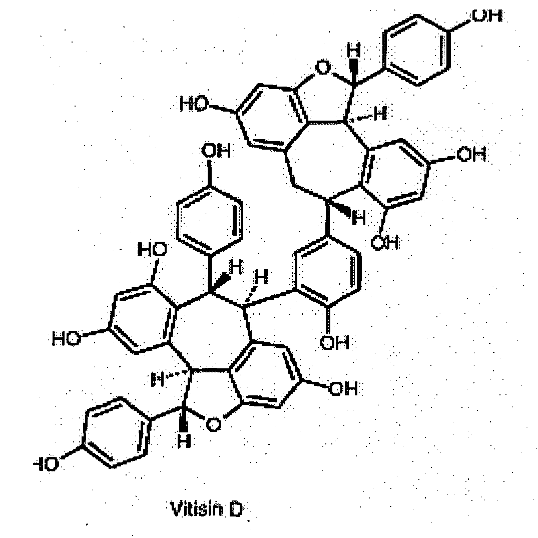 Vitisin D (RESV-IV)

Die Summenformel von trans-epsilon-Viniferin lautet C₂₈H₂₂O₆ (CAS: 62218-08-0). Man findet diese Substanz in freier Form oder als Glycosid in variablen Mengen in diversen Pflanzenarten, darunter Vitaceae, Umbelliferae, Myrtaceae, Dipterocarpaceae, Cyperaceae, Gnetaceae, Leguminosen, Gramineae, Sericeae, Haemodoraceae, Musaceae, Polygonaceae, Pinaceae, Cupressaceae, Cesalpiniaceae, Poaceae, et Solanaceae, insbesondere in Balanocarpus zeylanicus, Caragana chamlagu, Caragana sinica, Carex fedia, Carex humilis, Carex kobomugi, Carex pendula, Carex pumila Thunb, Cyphostemma crotalarioides, Gnetum hainanense, Gnetum ula, Gnetum venosum, Hopea parviflora, Iris clarkei, Neobalanocarpus heimii, Paeoni lactiflora, Parthenocissus tricuspidata, Polygonum cuspidatum, Polygonum multiflorum, Shorea disticha, Shorea hemsleyana, Sophora davidii, Sophora leachiana, Sophora nuttaliana, Vatica rassak, Vatica affinis, Vitis amurensis, Vitis betulifolia, Vitis flexuosa, Vitis heyneana, Vitis quinquangularis, Vitis coignetiae, Vitis vinifera. Im Stand der Technik bekannt ist die hormonähnliche Wirkung des trans-epsilon-Viniferins, die wiederum der Wirkung der Retinoide ähnelt.

Besonders bevorzugt werden die Hydroxystilben-Oligomere aus den Vitaceae extrahiert, insbesondere aus den Stielen von Weintrauben, da diese mengenmäßig gut verfügbar sind. Als Extraktionsmittel eignen sich sowohl Wasser als auch organische Lösemittel. Bevorzugte organische Lösemittel sind Ethylacetat und/oder Diethylether. Störende Lipide, die ebenfalls mit extrahiert werden, können beispielsweise mittels Petrolether, Hexan oder Chloroform entfernt werden. Bevorzugte Extraktionsverfahren erfolgen mit Hilfe organischer Lösemittel und Mikrowellen- oder Ultraschall-Behandlung, oder mittels Maceration-Lixiviation oder mittels Extraktion durch superkritische Fluide. Die so angereicherten Extrakte werden vorteilhafterweise filtriert, gewaschen und besonders bevorzugt gefriergetrocknet.

Unter den Oligomeren von Hydroxystilbenen werden erfindungsgemäß Dimere, Trimere, Tetramere, Pentamere und Hexamere von Hydroxystilbenen verstanden. Besonders bevorzugt sind die Dimere, Trimere und Tetramere, außerordentlich bevorzugt sind die Dimere von Hydroxystilbenen.

Die Oligomerisierung erfolgt erfindungsgemäß besonders bevorzugt durch den Angriff einer Hydroxylgruppe an die ethylenische Doppelbindung eines weiteren Hydroxystilben-Moleküls unter Ausbildung eines Dihydrofuranrings, wie es oben exemplarisch am Beispiel von (E)-Resveratrol und den daraus gebildeten Oligomeren trans-epsilon-Viniferin, Vitisin E und Vitisin D beschrieben ist.

Prinzipiell sind aber auch andere Oligomerisierungsreaktionen möglich und können bevorzugt sein, beispielsweise die Kondensation von zwei und mehr Hydroxystilben-Molekülen über die Hydroxylgruppen unter Bildung von Etherbindungen. Da die durch Kondensation gebildeten Oligomere sterisch weniger stark gehindert sind als die Oligomere, die an der ethylenischen Stilben-Doppelbindung entstehen, sind hierbei neben den Dimeren, Trimeren, Tetrameren, Pentameren und Hexameren auch höhere Oligomere, beispielsweise Heptamere und Octamere, möglich.

Die Hydroxystilben-Oligomere können aus gleichen Monomeren (= Homooligomerisierung) oder aus verschiedenen Monomeren (= Heterooligomerisierung) gebildet sein. Erfindungsgemäß bevorzugt sind die Homooligomere, bezogen auf die nicht-derivatisierten, das heißt beispielsweise, nicht veretherten oder nicht-veresterten, Hydroxystilben-Monomere.

Die Derivatisierung der Hydroxystilbene, beispielsweise die Veretherung oder Veresterung der OH-Gruppen, kann vor oder nach der Oligomerisierung erfolgen. Die Derivatisierung nach der Oligomerisierung kann zu einem unsymmetrischen Substitutionsmuster führen.

Erfindungsgemäß bevorzugt verwendete Ester der Hydroxystilben-Oligomere werden bevorzugt hergestellt nach einem der Verfahren, wie sie in der Offenlegungsschrift FR 2766176 A1 dargestellt sind, insbesondere die Verfahren zur Herstellung der Sorbinsäureester, Hexansäureester, Palmitinsäureester und Laurinsäureester.

Erfindungsgemäß besonders bevorzugt verwendete Hydroxystilben-Oligomere sind aus Hydroxystilben-Monomeren der allgemeinen Formel (RESV-I a) und (RESV-I b) gebildet, 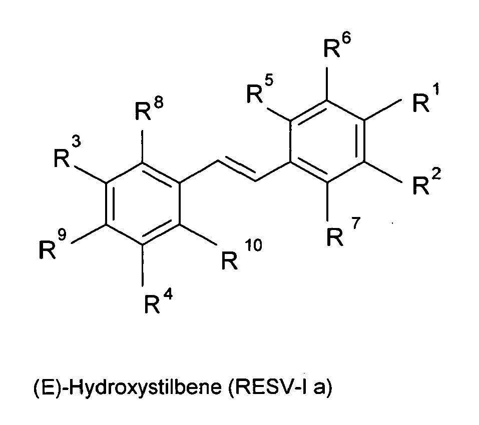 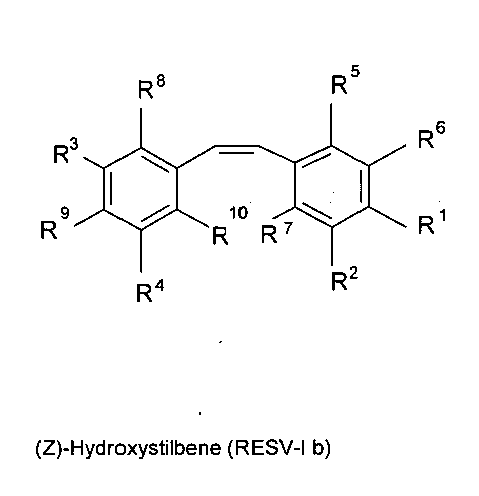 wobei die Reste R¹ bis R¹⁰ unabhängig voneinander ein Wasserstoffatom, eine OH-Gruppe, eine Alkylgruppe, die bevorzugt eine C₁-C₄-Alkylgruppe darstellt, eine Alkoxygruppe, die bevorzugt einen C₁-C₄-Alkylrest trägt, ein Glycosid-Rest, eine ganz oder teilweise neutralisierte Phosphatgruppe -OPO₃, eine ganz oder teilweise neutralisierte Sulfatgruppe -OSO₃ oder eine Estergruppe -OCOR' mit R'COOH = organische Säure sein können, wobei mindestens einer der Reste R¹ bis R¹⁰ ausgewählt ist aus einer OH-Gruppe, einer Alkoxygruppe, die bevorzugt einen C₁-C₄-Alkylrest trägt, einem Glycosid-Rest, einer ganz oder teilweise neutralisierten Phosphatgruppe -OPO₃, einer ganz oder teilweise neutralisierten Sulfatgruppe -OSO₃ und einer Estergruppe -OCOR' mit R'COOH = organische Säure.

Die Alkylgruppe in den Formeln RESV-I a und RESV-I b ist erfindungsgemäß bevorzugt ausgewählt aus einer Methyl-, Ethyl-, n-Propyl-, i-Propyl-, n-Butyl-, sec-Butyl-, tert.-Butyl-Gruppe und 2-Ethylhexyl-Gruppe, besonders bevorzugt ausgewählt aus einer Methyl-Gruppe.

Die Alkoxygruppe in den Formeln RESV-I a und RESV-I b ist erfindungsgemäß bevorzugt ausgewählt aus einer Methoxy-, Ethoxy-, n-Propoxy-, i-Propoxy-, n-Butoxy-, sec-Butoxy-, tert.-Butoxy-Gruppe und 2-Ethylhexoxy-Gruppe, besonders bevorzugt ausgewählt aus einer Methoxy-Gruppe. Der Glycosid-Rest in den Formeln RESV-I a und RESV-I b ist erfindungsgemäß bevorzugt ausgewählt aus einem Rest, abstammend von D-Glucose, Fructose, D-Galactose, L-Arabinose, Ribose, D-Xylose, Lyxose, Allose, Altrose, Mannose, Gulose, Idose, Talose, Fucose, und Sucrose, wobei D-Glucose, D-Apiose, L-Rhamnose, L-Rhamnoglycosiden, Rutinose (6-O-alpha-L-Rhamnopyranosyl-D-glucose), D-Glucuronsäure, D-Galacturonsäure und Neohesperidose besonders bevorzugt sind.

Die organische Säure R'COOH, die zur Veresterung der in den Formeln RESV-I a und RESV-I b dargestellten Verbindungen verwendet wird, ist erfindungsgemäß bevorzugt ausgewählt aus den linearen und verzweigten, gesättigten, einfach ungesättigten und mehrfach ungesättigten, ggf. aromatischen C₁-C₃₀-Monocarbonsäuren, insbesondere Ameisensäure, Essigsäure, Propansäure, Butansäure, Pentansäure, Valeriansäure, Isovaleriansäure, Hexansäure, Sorbinsäure ((*E,E*)-2,4-Hexadiensäure), 2-Ethylhexansäure, Heptansäure, Benzoesäure, Octansäure, Nonansäure, Decansäure, Undecansäure, Laurinsäure, Tridecansäure, Tetradecansäure, Myristinsäure, Palmitinsäure, Palmitoleinsäure, Stearinsäure, Ölsäure, Elaidinsäure, Petroselinsäure ((Z)-6-Octadecensäure), Linolsäure, Linolensäure, Arachinsäure, Arachidonsäure, (all-Z)-5,8,11,14,17-Eicosapentaensäure, Behensäure, Erucasäure, (all-Z)-4,7,10,13,16,19-Docosahexaensäure, weiterhin bevorzugt ausgewählt aus den linearen und verzweigten, gesättigten, einfach ungesättigten und mehrfach ungesättigten, ggf. aromatischen C₂-C₂₀-Hydroxymono-, -di- und tricarbonsäuren, insbesondere Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Ricinolsäure, Mandelsäure (Hydroxyphenylessigsäure), 4-Hydroxymandelsäure, Äpfelsäure (Hydroxybernsteinsäure), Erythrarsäure, Threarsäure, Glucarsäure, Galactarsäure, Mannarsäure, Gularsäure, 2-Hydroxy-2-methylbernsteinsäure, Gluconsäure, Glucuronsäure, Galacturonsäure und Salicylsäure, wobei Hexansäure, Sorbinsäure ((*E,E*)-2,4-Hexadiensäure), 2-Ethylhexansäure, Octansäure, Decansäure, Laurinsäure, Myristinsäure, Palmitinsäure, Stearinsäure, Glycolsäure und Milchsäure besonders bevorzugt sind.

Erfindungsgemäß besonders bevorzugt sind die Oligomere der (E)-Hydroxystilbene, insbesondere die Dimere der (E)-Hydroxystilbene.

Erfindungsgemäß besonders bevorzugt verwendete Hydroxystilben-Oligomere sind aus folgenden Hydroxystilben-Monomeren gebildet:
4'-Hydroxystilben (R¹ = OH),
2',4'-Dihydroxystilben (R¹ = R⁵ = OH),
3',4'-Dihydroxystilben (R² = R⁵ = OH),
4,4'-Dihydroxystilben (R¹ = R⁹ = OH),
3,5- Dihydroxystilben (Pinosylvin) (R³ = R⁴ = OH),
3-Hydroxy-5-methoxystilben (Pinosylvinmonomethylether, (R³ = OH, R⁴ = -OCH₃)
2',4',4-Trihydroxystilben (R¹ = R⁵ = R⁹ = OH),
3',4',4-Trihydroxystilben (R² = R⁵ = R⁹ = OH),
2,4,4'-Trihydroxystilben (R¹ = R⁸ = R⁹ = OH),
3,4,4'-Trihydroxystilben (R¹ = R³ = R⁹ = OH),
3,4',5-Trihydroxystilben (Resveratrol) (R¹ = R³ = R⁴ = OH),
2',3,4-Trihydroxystilben (R¹ = R⁵ = R⁶ = OH),
2,3',4-Trihydroxystilben (R⁵ = R⁶ = _{R}⁸ = OH),
2',2,4'-Trihydroxystilben (R¹ = R⁵ = R⁸ = OH),
2,4,4',5-Tetrahydroxystilben (R¹ = R⁴ R⁸ _{R}⁹ = OH),
2',3,4',5-Tetrahydroxystilben (R¹ =R³ = R⁴ = R⁵ = OH),
2,2',4,4'-Tetrahydroxystilben (R¹ R⁵ R⁸ = R⁹ = OH),
3,3',4',5-Tetrahydroxystilben (Piceatannol) (R¹ = R³ = R⁴ = R⁶ = OH),
2,3',4,4'-Tetrahydroxystilben (R¹ = R⁶ = R⁸ = R⁹ = OH),
3,3',4,4'-Tetrahydroxystilben (R¹ = R³ = R⁶ = R⁹ = OH),
3,3',4',5,5'-Pentahydroxystilben (R¹ = R² = R³ = R⁴ = R⁶ = OH),
2,2',4,4',6-Pentahydroxystilben (R¹ = R⁵ = R⁸ = R⁹ = R¹⁰ = OH),
2,3',4,4',6-Pentahydroxystilben (R¹ = R⁶ R⁸ R⁹ = R¹⁰ = OH),
2,2',4,4',6,6'-Hexahydroxystilben (R¹ = R⁵ = R⁷ = R⁸ = R⁹ = R¹⁰ = OH),
wobei für die nicht genannten R^{x} = H gilt, das heißt, alle übrigen Substituenten R stellen ein Wasserstoffatom dar, und wobei alle Verbindungen sowohl in der (E)-Form als auch in der (Z)-Form vorliegen, wobei die Verbindungen, die in der (E)-Form vorliegen, besonders bevorzugt sind.

Erfindungsgemäß besonders bevorzugt verwendete Hydroxystilben-Oligomere sind ausgewählt aus trans-epsilon-Viniferin, Vitisin E und Vitisin D sowie Mischungen hiervon, weiterhin ausgewählt aus Mono-, Di-, Tri-, Tetra- und Pentamethoxy-trans-epsilon-Viniferin, trans-epsilon-Viniferin-Mono-, Di-, Tri-, Tetra- und Pentaalkanoaten, insbesondere trans-epsilon-Viniferin-Mono-, Di-, Tri-, Tetra- und Pentapalmitat, Mono-, Di-, Tri-, Tetra-, Penta-, Hexa- und Heptamethoxy-Vitisin E, Vitisin E-Mono-, Di-, Tri-, Tetra-, Penta-, Hexa- und Heptaalkanoaten, insbesondere Vitisin E-Mono-, Di-, Tri-, Tetra-, Penta-, Hexa- und Heptapalmitat, Mono-, Di-, Tri-, Tetra-, Penta-, Hexa-, Hepta-, Octa-, Nona- und Decamethoxy-Vitisin D, Vitisin D-Mono-, Di-, Tri-, Tetra-, Penta-, Hexa-, Hepta-, Octa-, Nona- und Decaalkanoaten, insbesondere Vitisin D-Mono-, Di-, Tri-, Tetra-, Penta-, Hexa-, Hepta-, Octa-, Nona- und Decapalmitat, sowie Mischungen hiervon.

Ein weiterer Gegenstand der vorliegenden Erfindung sind Zusammensetzungen, insbesondere kosmetische oder dermatologische Zusammensetzungen, die zur topischen Behandlung der Haut geeignet sind, die in einem für den jeweiligen Anwendungszweck geeigneten Träger mindestens ein Hydroxystilben-Oligomer oder einen Alkylether hiervon oder einen Ester hiervon in Kombination mit mindestens einem Aporphin-Alkaloid enthalten.

Überraschend wurde festgestellt, dass die Wirkung des erfindungsgemäß verwendeten Hydroxystilben-Oligomers in unerwarteter Weise durch ein Aporphin-Alkaloid gesteigert werden kann. Ohne an diese Theorie gebunden sein zu wollen, wird vermutet, dass der Zusatz von mindestens einem Aporphin-Alkaloid zu einer optimierten Regulierung der Elektrolytversorgung auf zellulärer Ebene führt, wodurch der Übergang der Zellen, insbesondere der Zellen der Epidermis und/oder der Dermis aus dem proliferativen Status in den ruhenden, seneszenten Status noch stärker verzögert, und/oder der Anteil der Zellen, insbesondere der Zellen in der Epidermis und/oder der Dermis, die sich im ruhenden, seneszenten Status befinden, weiter verringert wird.

Erfindungsgemäß bevorzugte Aporphin-Alkaloide sind ausgewählt ist aus Verbindungen der allgemeinen Struktur (APO-ALK-1), 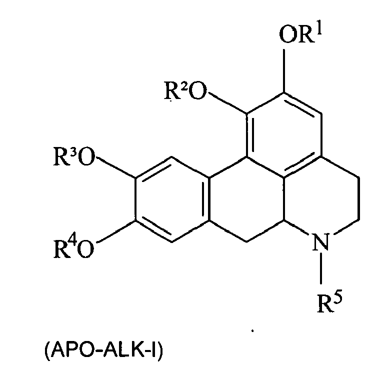 in der die Reste R¹, R², R³, R⁴ und R⁵, die gleich oder verschieden sein können, ausgewählt sind aus einem Wasserstoffatom, einem Halogenatom, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe, einer Aryl-C₁-C₆-alkylgruppe, einer Acylgruppe, einer Sulfonylgruppe oder einem Zucker, und den kosmetisch verträglichen Salzen von (APO-ALK-I) mit einer Säure, in Form aller 15 optischen Isomere, in isomerenreiner Form oder als Mischung optischer Isomere.

Beispiele für die als Substituenten in den erfindungsgemäß kombinierten Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt.

Als Zuckerrest können beliebige Mono- oder Oligo-, insbesondere Disaccharide, eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Besonders bevorzugte Aporphin-Alkaloide sind ausgewählt aus Verbindungen der allgemeinen Struktur (APO-ALK-I), in denen R¹ = R² = R³ = R⁴ = R⁵ = CH₃ ist. Diese Verbindung wird auch als 1,2,9,10-Tetramethoxyaporphin bezeichnet.

Besonders bevorzugte erfindungsgemäße Zusammensetzungen enthalten mindestens ein Aporphin-Alkaloid in engeren Mengenbereichen. Hier sind erfindungsgemäß bevorzugte Zusammensetzungen, insbesondere kosmetische Zusammensetzungen, dadurch gekennzeichnet, dass sie - bezogen auf ihr Gewicht - eine Gesamtmenge von 0,0001 - 1 Gew.-%, vorzugsweise 0,001 - 0,5 Gew.-%, besonders bevorzugt 0,002 - 0,1 Gew.-% und insbesondere 0,005 - 0,01 Gew.-% mindestens eines Aporphin-Alkaloids enthalten.

Erfindungsgemäß besonders bevorzugte Zusammensetzungen sind gekennzeichnet durch einen Gehalt an trans-epsilon-Viniferin und 1,2,9,10-Tetramethoxyaporphin.

Weitere erfindungsgemäß besonders bevorzugte Zusammensetzungen sind gekennzeichnet durch einen Gehalt an Vitisin E und 1,2,9,10-Tetramethoxyaporphin.

Weitere erfindungsgemäß besonders bevorzugte Zusammensetzungen sind gekennzeichnet durch einen Gehalt an Vitisin D und 1,2,9,10-Tetramethoxyaporphin.

Es ist weiterhin bevorzugt, dass in den vorstehend genannten Zusammensetzungen ein Alkylether des (E)-Hydroxystilben-Oligomers mit dem 1,2,9,10-Tetramethoxyaporphin kombiniert wird. Es ist weiterhin bevorzugt, dass in den vorstehend genannten Zusammensetzungen ein Ester des (E)-Hydroxystilben-Oligomers mit dem 1,2,9,10-Tetramethoxyaporphin kombiniert wird.

Ein weiterer Gegenstand der vorliegenden Erfindung sind Zusammensetzungen, insbesondere kosmetische oder dermatologische Zusammensetzungen, die insbesondere zur topischen Behandlung der Haut geeignet sind, die in einem für den jeweiligen Anwendungszweck geeigneten Träger mindestens ein Hydroxystilben-Oligomer oder einen Alkylether hiervon oder einen Ester hiervon in Kombination mit mindestens einem Wirkstoff enthalten, der ausgewählt ist aus Purin und den Derivaten des Purins. Purin (7*H*-Imidazo[4,5-*d*]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden. Purine und Purinderivate werden oft aus Naturstoffen isoliert, sind aber auch auf vielen Wegen synthetisch zugänglich.

Überraschend wurde festgestellt, dass die Wirkung des erfindungsgemäß verwendeten Hydroxystilben-Oligomers in unerwarteter Weise durch Purin und/oder Purinderivat(e) gesteigert werden kann. Ohne an diese Theorie gebinden sein zu wollen, wird vermutet, dass der Zusatz von Purin und/oder mindestens einem Purinderivat zu einer optimierten Regulierung der Nährstoffversorgung, insbesondere derLipidversorgung, auf zellulärer Ebene führt, wodurch der Übergang der Zellen, insbesondere der Zellen der Epidermis und/oder der Dermis aus dem proliferativen Status in den ruhenden, seneszenten Status noch stärker verzögert, und/oder der Anteil der Zellen, insbesondere der Zellen in der Epidermis und/oder der Dermis, die sich im ruhenden, seneszenten Status befinden, weiter verringert wird.

Besonders bevorzugte erfindungsgemäße Zusammensetzungen enthalten Purin und/oder Purinderivat(e) in engeren Mengenbereichen. Hier sind erfindungsgemäß bevorzugte Zusammensetzungen, insbesondere kosmetische Zusammensetzungen, dadurch gekennzeichnet, dass sie - bezogen auf ihr Gewicht - eine Gesamtmenge von 0,001 - 2,5 Gew.-%, vorzugsweise 0,01 - 1 Gew.-%, besonders bevorzugt 0,1 - 0,8 Gew.-% und insbesondere 0,2 - 0,5 Gew.-% Purin(e) und/oder Purinderivat(e) enthalten.

Unter Purin, den Purinen und den Purinderivaten sind einige Vertreter erfindungsgemäß besonders bevorzugt. Erfindungsgemäß bevorzugte Zusammensetzungen sind dadurch gekennzeichnet, dass sie Purin und/oder Purinderivat(e) der Formel (PUR-I) enthalten,

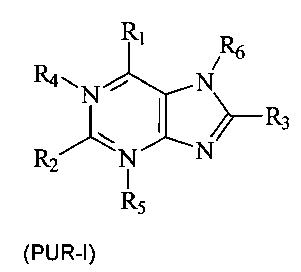

in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁- bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

Erfindungsgemäß besonders bevorzugte Zusammensetzungen sind dadurch gekennzeichnet, dass sie Purin und/oder Purinderivat(e) der Formel (PUR-1) enthalten, in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, -CH₃ und -CH₂-CH₃, wobei folgende Verbindungen außerordentlich bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R²=OH, R³ = H, R⁴ = CH₃, R⁵ = CH₃, R⁶ = H).

Verbindungen der allgemeinen Formel (PUR-l), in denen R¹ und/oder R² einer OH-Gruppe entsprechen, existieren in tautomeren Verbindungen, insbesondere solchen der allgemeinen Formel (PUR-II), ausgehend von (PUR-I) mit R¹ = R² = OH : 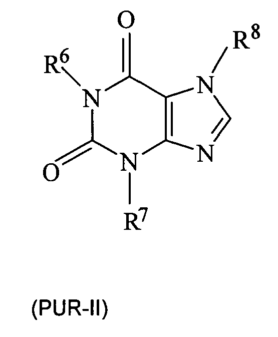 wobei in der Formel (PUR-II) die Reste R⁶, R⁷ und R⁸, die gleich oder verschieden sein können, ausgewählt sind aus einem Wasserstoffatom, einem C₁- bis C₄-Alkylrest, einem C₁- bis C₄-Mono-hydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe, wobei Coffein (R⁶ = R⁷ = R⁸ = CH₃), Theobromin (R⁶ = H, R⁷ = R⁸ = CH₃) und Theophyllin (R⁶ = R⁷ = CH₃, R⁸ = H) außerordentlich bevorzugt sind.

Je nach gewünschtem Anwendungszweck der erfindungsgemäßen Zusammensetzung kann dabei die Art und Menge des Purinderivates variieren. In haarkosmetischen Formulierungen und in Körperpflegemitteln zur Hautbehandlung außerhalb des Gesichts hat sich insbesondere Coffein bewährt, das in den genannten Mitteln, insbesondere Cremes, Gelen und Lotionen, vorzugsweise in Mengen von 0,01 - 2 Gew.-%, weiter bevorzugt 0,1 - 1,5 Gew.-% und besonders bevorzugt 0,2 - 0,8 Gew.-%, jeweils bezogen auf das Mittel, eingesetzt werden kann.

Allgemein sind erfindungsgemäß Zusammensetzungen bevorzugt, die Purin und/oder mindestens ein Purinderivat in einer Gesamtmenge von 0,0001 - 2 Gew.-%, bevorzugt 0,001 - 1,5 Gew.-% besonders bevorzugt 0,05 - 1 Gew.-%, undaußerordentlich bevorzugt 0,1 - 0,5 Gew.-%, jeweils bezogen auf die Zusammensetzung, enthalten.

Erfindungsgemäß besonders bevorzugte Zusammensetzungen sind gekennzeichnet durch einen Gehalt an trans-epsilon-Viniferin und Coffein.

Weitere erfindungsgemäß besonders bevorzugte Zusammensetzungen sind gekennzeichnet durch einen Gehalt an Vitisin E und Coffein.

Weitere erfindungsgemäß besonders bevorzugte Zusammensetzungen sind gekennzeichnet durch einen Gehalt an Vitisin D und Coffein.

Weitere erfindungsgemäß besonders bevorzugte Zusammensetzungen sind gekennzeichnet durch einen Gehalt an trans-epsilon-Viniferin und Theobromin.

Weitere erfindungsgemäß besonders bevorzugte Zusammensetzungen sind gekennzeichnet durch einen Gehalt an Vitisin E und Theobromin.

Weitere erfindungsgemäß besonders bevorzugte Zusammensetzungen sind gekennzeichnet durch einen Gehalt an Vitisin D und Theobromin.

Weitere erfindungsgemäß besonders bevorzugte Zusammensetzungen sind gekennzeichnet durch einen Gehalt an trans-epsilon-Viniferin und Theophyllin.

Weitere erfindungsgemäß besonders bevorzugte Zusammensetzungen sind gekennzeichnet durch einen Gehalt an Vitisin E und Theophyllin.

Weitere erfindungsgemäß besonders bevorzugte Zusammensetzungen sind gekennzeichnet durch einen Gehalt an Vitisin D und Theophyllin.

Es ist weiterhin bevorzugt, dass in den vorstehend genannten Zusammensetzungen ein Alkylether des (E)-Hydroxystilben-Oligomers mit Coffein, Theobromin oder Theophyllin kombiniert wird.

Es ist weiterhin bevorzugt, dass in den vorstehend genannten Zusammensetzungen ein Ester des (E)-Hydroxystilben-Oligomers mit Coffein, Theobromin oder Theophyllin kombiniert wird.

Ein weiterer Gegenstand der vorliegenden Erfindung sind Zusammensetzungen, insbesondere kosmetische oder dermatologische Zusammensetzungen, die zur topischen Behandlung der Haut geeignet sind, die in einem für den jeweiligen Anwendungszweck geeigneten Träger mindestens einen Wirkstoff, ausgewählt aus Hydroxystilben-Oligomeren, Alkylethern hiervon und Estern hiervon, in Kombination mit mindestens einer natürlichen Betainverbindung enthalten.

Überraschend wurde festgestellt, dass die Wirkung des erfindungsgemäß verwendeten Hydroxystilben-Oligomers in unerwarteter Weise durch eine natürliche Betainverbindung gesteigert werden kann. Ohne an diese Theorie gebunden sein zu wollen, wird vermutet, dass der Zusatz von mindestens einer natürlichen Betainverbindung zu einer optimierten Regulierung der Nährstoffversorgung auf zellulärer Ebene und/oder innerhalb der extracellulären Matrix (ECM) führt, wodurch die Wirkung der erfindungsgemäß verwendeten Hydroxystilben-Oligomere synergistisch unterstützt werden kann und wodurch der Übergang der Zellen, insbesondere der Zellen der Epidermis und/oder der Dermis aus dem proliferativen Status in den ruhenden, seneszenten Status noch stärker verzögert, und/oder der Anteil der Zellen, insbesondere der Zellen in der Epidermis und/oder der Dermis, die sich im ruhenden, seneszenten Status befinden, weiter verringert werden kann.

Erfindungsgemäß bevorzugte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻), Carnitin (Me₃N⁺-CH₂-CHOH-CH₂-COO⁻), jeweils mit Me = Methyl, und Ergothionein = 2-Mercapto-N^{α}N^{α}N^{α}-trimethyl-L-histidinium-Betain der Formel (BETA-II)

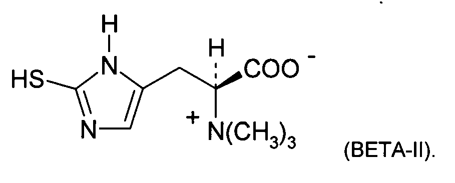

Die Herkunft der eingesetzten natürlichen Betainverbindung kann erfindungsgemäß durchaus synthetisch sein.

Weiterhin können auch Salze und/oder Ester der natürlichen Betainverbindungen erfindungsgemäß bevorzugt eingesetzt werden. Ein derartiges besonders bevorzugtes Derivat ist Carnitintartrat.

Bevorzugte erfindungsgemäße Zusammensetzungen sind dadurch gekennzeichnet, dass sie zusätzlich zu dem mindestens einen erfindungsgemäß verwendeten Hydroxystilben-Oligomer-(derivat) mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte Zusammensetzung, enthalten.

Erfindungsgemäß besonders bevorzugte Zusammensetzungen sind gekennzeichnet durch einen Gehalt an trans-epsilon-Viniferin und Betain.

Weitere erfindungsgemäß besonders bevorzugte Zusammensetzungen sind gekennzeichnet durch einen Gehalt an Vitisin E und Betain.

Weitere erfindungsgemäß besonders bevorzugte Zusammensetzungen sind gekennzeichnet durch einen Gehalt an Vitisin D und Betain.

Weitere erfindungsgemäß besonders bevorzugte Zusammensetzungen sind gekennzeichnet durch einen Gehalt an trans-epsilon-Viniferin und Carnitin.

Weitere erfindungsgemäß besonders bevorzugte Zusammensetzungen sind gekennzeichnet durch einen Gehalt an Vitisin E und Carnitin.

Weitere erfindungsgemäß besonders bevorzugte Zusammensetzungen sind gekennzeichnet durch einen Gehalt an Vitisin D und Carnitin.

Weitere erfindungsgemäß besonders bevorzugte Zusammensetzungen sind gekennzeichnet durch einen Gehalt an trans-epsilon-Viniferin und Carnitintartrat.

Weitere erfindungsgemäß besonders bevorzugte Zusammensetzungen sind gekennzeichnet durch einen Gehalt an Vitisin E und Carnitintartrat.

Weitere erfindungsgemäß besonders bevorzugte Zusammensetzungen sind gekennzeichnet durch einen Gehalt an Vitisin D und Carnitintartrat.

Erfindungsgemäß besonders bevorzugte Zusammensetzungen sind gekennzeichnet durch einen Gehalt an trans-epsilon-Viniferin und Ergothionein.

Weitere erfindungsgemäß besonders bevorzugte Zusammensetzungen sind gekennzeichnet durch einen Gehalt an Vitisin E und Ergothionein.

Weitere erfindungsgemäß besonders bevorzugte Zusammensetzungen sind gekennzeichnet durch einen Gehalt an Vitisin D und Ergothionein.

Es ist weiterhin bevorzugt, dass in den vorstehend genannten Zusammensetzungen ein Alkylether des (E)-Hydroxystilben-Oligomers mit Betain, Carnitin, Carnitintartrat oder Ergothionein kombiniert wird.

Es ist weiterhin bevorzugt, dass in den vorstehend genannten Zusammensetzungen ein Ester des (E)-Hydroxystilben-Oligomers mit Betain, Carnitin, Carnitintartrat oder Ergothionein kombiniert wird.

Ein weiterer Gegenstand der vorliegenden Erfindung sind Zusammensetzungen, insbesondere kosmetische oder dermatologische Zusammensetzungen, die insbesondere zur topischen Behandlung der Haut geeignet sind, die in einem geeigneten Träger mindestens einen Wirkstoff, ausgewählt aus Hydroxystilben-Oligomeren, Alkylethern hiervon und Estern hiervon, in Kombination mit mindestens einer Substanz enthalten, die ausgewählt ist aus Harnstoff und alkyl- oder hydroxyalkylsubstituierten Harnstoffen.

Überraschend wurde festgestellt, dass die Wirkung des erfindungsgemäß verwendeten Hydroxystilben-Oligomer(derivat)s in unerwarteter Weise durch Substanzen, ausgewählt aus Harnstoff und alkyl- oder hydroxyalkylsubstituierten Harnstoffen, gesteigert werden kann. Ohne an diese Theorie gebunden sein zu wollen, wird vermutet, dass der Zusatz von mindestens einer Substanz, ausgewählt aus Harnstoff und alkyl- oder hydroxyalkylsubstituierten Harnstoffen, zu einer optimierten Regulierung der Nährstoffversorgung innerhalb der extracellulären Matrix (ECM) führt, wodurch die Wirkung der erfindungsgemäß verwendeten Hydroxystilben-Oligomere synergistisch unterstützt werden kann und wodurch der Übergang der Zellen, insbesondere der Zellen der Epidermis und/oder der Dermis aus dem proliferativen Status in den ruhenden, seneszenten Status noch stärker verzögert, und/oder der Anteil der Zellen, insbesondere der Zellen in der Epidermis und/oder der Dermis, die sich im ruhenden, seneszenten Status befinden, weiter verringert werden kann.

Erfindungsgemäß bevorzugte alkyl- oder hydroxyalkylsubstituierte Harnstoffverbindungen weisen die allgemeine Formel (UREA-1) auf, 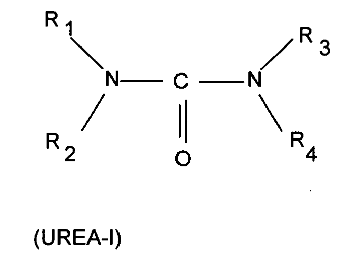 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Erfindungsgemäß besonders bevorzugte alkyl- oder hydroxyalkylsubstituierte Harnstoffverbindungen der allgemeinen Formel (UREA-I) sind ausgewählt aus Verbindungen, bei denen jeweils mindestens einer der Reste R₁ und R₂ bzw. R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Besonders bevorzugte C₂-C₆-Hydroxyalkyl-Gruppen, die mit 1 bis 5 Hydroxylgruppen oder C₁-C₄-HydroxyalkylGruppen substituiert sind, sind ausgewählt aus 2-Hydroxyethyl-, 3-Hydroxypropyl-, 2-Hydroxypropyl, 2-Hydroxyisopropyl- und 4-Hydroxybutyl-Gruppen, wobei die 2-Hydroxyethyl-Gruppe außerordentlich bevorzugt ist. Ebenfalls außerordentlich bevorzugt ist Bis-N,N'-(2-Hydroxyethyl)-harnstoff. Harnstoff selbst ist ebenfalls besonders bevorzugt. Weiterhin bevorzugt ist es, Mischungen von Harnstoff und alkyl- oder hydroxyalkylsubstituierten Harnstoffverbindungen einzusetzen. Außerordentlich bevorzugt sind Mischungen von Harnstoff und Bis-N,N'-(2-Hydroxyethyl)-harnstoff.

Bevorzugte erfindungsgemäße Zusammensetzungen sind dadurch gekennzeichnet, dass sie zusätzlich zu dem mindestens einen erfindungsgemäß verwendeten Hydroxystilben-Oligomer-(derivat) mindestens eine Substanz, ausgewählt aus Harnstoff und alkyl- oder hydroxyalkylsubstituierten Harnstoffen, in einer Gesamtmenge von 0,01 bis 10 Gew.-%, bevorzugt 0,1 bis 5 Gew.-%, besonders bevorzugt 0,2 bis 3 Gew.-% und außerordentlich bevorzugt 0,5 - 1 Gew.-%, jeweils bezogen auf die gesamte Zusammensetzung, enthalten.

Die handelsüblich erhältlichen alkyl- oder hydroxyalkylsubstituierten Harnstoff-Zubereitungen enthalten meistens als Neben- oder Abbauprodukt unsubstituierten Harnstoff. Daher sind erfindungsgemäß auch Kombinationen aus Harnstoff und mindestens einer Substanz, ausgewählt aus alkyl- oder hydroxyalkylsubstituierten Harnstoffen, bevorzugt.

Erfindungsgemäß besonders bevorzugte Zusammensetzungen sind gekennzeichnet durch einen Gehalt an trans-epsilon-Viniferin und Bis-N,N'-(2-Hydroxyethyl)-harnstoff.

Weitere erfindungsgemäß besonders bevorzugte Zusammensetzungen sind gekennzeichnet durch einen Gehalt an Vitisin E und Bis-N,N'-(2-Hydroxyethyl)-harnstoff.

Weitere erfindungsgemäß besonders bevorzugte Zusammensetzungen sind gekennzeichnet durch einen Gehalt an Vitisin D und Bis-N,N'-(2-Hydroxyethyl)-harnstoff.

Weitere erfindungsgemäß besonders bevorzugte Zusammensetzungen sind gekennzeichnet durch einen Gehalt an trans-epsilon-Viniferin und Harnstoff.

Weitere erfindungsgemäß besonders bevorzugte Zusammensetzungen sind gekennzeichnet durch einen Gehalt an Vitisin E und Harnstoff.

Weitere erfindungsgemäß besonders bevorzugte Zusammensetzungen sind gekennzeichnet durch einen Gehalt an Vitisin D und Harnstoff.

Weitere erfindungsgemäß besonders bevorzugte Zusammensetzungen sind gekennzeichnet durch einen Gehalt an trans-epsilon-Viniferin, Harnstoff und Bis-N,N'-(2-Hydroxyethyl)-harnstoff. Weitere erfindungsgemäß besonders bevorzugte Zusammensetzungen sind gekennzeichnet durch einen Gehalt an Vitisin E, Harnstoff und Bis-N,N'-(2-Hydroxyethyl)-harnstoff.

Weitere erfindungsgemäß besonders bevorzugte Zusammensetzungen sind gekennzeichnet durch einen Gehalt an Vitisin D, Harnstoff und Bis-N,N'-(2-Hydroxyethyl)-harnstoff.

Es ist weiterhin bevorzugt, dass in den vorstehend genannten Zusammensetzungen ein Alkylether des (E)-Hydroxystilben-Oligomers mit Harnstoff und/oder Bis-N,N'-(2-Hydroxyethyl)-harnstoff kombiniert wird.

Es ist weiterhin bevorzugt, dass in den vorstehend genannten Zusammensetzungen ein Ester des (E)-Hydroxystilben-Oligomers mit Harnstoff und/oder Bis-N,N'-(2-Hydroxyethyl)-harnstoff kombiniert wird.

Ein weiterer Gegenstand der vorliegenden Erfindung sind Zusammensetzungen, insbesondere kosmetische oder dermatologische Zusammensetzungen, die insbesondere zur topischen Behandlung der Haut geeignet sind, die in einem geeigneten Träger mindestens einen Wirkstoff, ausgewählt aus Hydroxystilben-Oligomeren, Alkylethern hiervon und Estern hiervon, in Kombination mit mindestens einer Substanz enthalten, die ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe. Überraschend wurde festgestellt, dass die Wirkung des erfindungsgemäß verwendeten Hydroxystilben-Oligomer(derivat)s in unerwarteter Weise durch Substanzen, ausgewählt aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe, gesteigert werden kann. Ohne an diese Theorie gebunden sein zu wollen, wird vermutet, dass der Zusatz von mindestens einer Substanz, ausgewählt aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe, zu einer optimierten Regulierung der Nährstoffversorgung auf zellulärer Ebene und/oder zu einer optimierten Regulierung der Nährstoffversorgung innerhalb der extracellulären Matrix (ECM) und/oder zu einer Stimulierung der Aufbauprozesse der extracellulären Matrix (ECM) führt, wodurch die Wirkung der erfindungsgemäß verwendeten Hydroxystilben-Oligomere synergistisch unterstützt werden kann und wodurch der Übergang der Zellen, insbesondere der Zellen der Epidermis und/oder der Dermis aus dem proliferativen Status in den ruhenden, seneszenten Status noch stärker verzögert, und/oder der Anteil der Zellen, insbesondere der Zellen in der Epidermis und/oder der Dermis, die sich im ruhenden, seneszenten Status befinden, weiter verringert werden kann.

Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Zink- und Natriumpyroglutamat und Natriumlauroylglutamat.

Der C₂ - C₂₄-Acylrest, mit dem die genannten Aminosäuren an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.

Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.

Die physiologisch verträglichen Salze der erfindungsgemäß bevorzugten Aminosäuren oder Aminosäurederivate sind bevorzugt ausgewählt aus den Ammonium-, Alkalimetall-, Magnesium-, Calcium-, Aluminium-, Zink- und Mangan-Salzen. Besonders bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäu-ren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen.

Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und IIe geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn, Gly-Asp-Ser, N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).

Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.

Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.

Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.

Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Glu-Glu-Met-Gln-Arg-Arg, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z..B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1 ). Ein weiteres bevorzugtes Aminosäureoligomer ist das Peptidderivat L-Glutamylaminoethyl-indol (enthalten in dem Rohstoff Glistin von Exsymol).

Ein erfindungsgemäß besonders bevorzugter Aminosäureoligomer-Wirkstoff ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Ein weiterer erfindungsgemäß bevorzugter Wirkstoff, der in Gegenwart von dem mindestens einen erfindungsgemäß verwendeten Wirkstoff, ausgewählt aus Hydroxystilben-Oligomeren, Alkylethern hiervon und Estern hiervon, eine unerwartete Wirkungssteigerung zeigt, ist ausgewählt aus Polymeren der Aminosäuren und/oder der N-C₂-C₂₄-Acylaminosäuren.

Erfindungsgemäß kombinierte Polymere der Aminosäuren und/oder der N-C₂-C₂₄-Acylamino-säuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich. mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.

Ein erfindungsgemäß besonders bevorzugtes Sojaproteinhydrolysat mit einem mittleren Molekulargewicht (Mw) im Bereich von 1200 - 1800 Dalton, bevorzugt im Bereich von 1400 - 1700 Dalton (Da), ist unter dem Handelsnamen Ridulisse C von der Firma Silab erhältlich. Der Proteinhydrolysat-Anteil in Ridulisse C enthält vier Molekulargewichtsfraktionen: 0,8 Gew.-% mit einem Mw > 12.500 Da, 22,2 Gew.-% mit 1355 Da < Mw < 12.500 Da, 43,1 Gew.-% 75 Da < Mw < 1355 Da und 33,9 Gew.-% < 75 Da.

Ein weiteres, erfindungsgemäß besonders bevorzugtes Sojaproteinhydrolysat ist ein mit Kokosfettsäuren N-acyliertes und/oder verestertes Sojaproteinhydrolysat in Form des Kaliumsalzes, das unter der Handelsbezeichnung Coccopolipeptide di Soja von der Firma Sinerga erhältlich ist.

Weiterhin erfindungsgemäß bevorzugt sind Keratinhydrolysate, insbesondere Wollkeratinhydrolysate. Ein besonders bevorzugtes Wollkeratinhydrolysat ist unter der Bezeichnung Keratec Pep von der Firma Croda erhältlich. Keratec Pep weist eine kleinere Molekulargewichtsfraktion mit einem durchschnittlichen Molekulargewicht von 150 Dalton und eine größere Molekulargewichtsfraktion mit einem durchschnittlichen Molekulargewicht von 1265 Dalton auf. Weiterhin erfindungsgemäß bevorzugt sind Conchiolinhydrolysate, insbesondere solche, die unter den Bezeichnungen Pearl Protein Extract und Pearl Protein Extract BG von der Firma Maruzen erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet.

Proteinhydrolysate können naturgemäß auch monomere Aminosäuren und Oligopeptide enthalten; ihre Zusammensetzung ist normalerweise nicht definiert.

Ein erfindungsgemäß besonders bevorzugtes Weizenproteinhydrolysat ist unter dem Handelsnamen Liftiline von der Firma Silab erhältlich.

Ebenfalls bevorzugt ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).

Erfindungsgemäß bevorzugt sind auch kationisierte Proteinhydrolysate. Besondes bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCl- Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.

In einer weiteren bevorzugten Ausführungsform sind die erfindungsgemäß kombinierten Polymeren der Aminosäuren ausgewählt aus DNA-Reparaturenzymen.

Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.

Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.

Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome^{™}, liposomenverkapselte T4N5 z. B. unter der Bezeichnung Ultrasome^{™} von der Firma AGI Dermatics, USA, erhältlich.

Erfindungsgemäß besonders bevorzugte Zusammensetzungen sind dadurch gekennzeichnet, dass sie neben dem mindestens einen erfindungsgemäß verwendeten Wirkstoff, ausgewählt aus Hydroxystilben-Oligomeren, Alkylethern hiervon und Estern hiervon, mindestens eines der Handelsprodukte Photosomes^{™} oder Ultrasomes^{™} in Gesamtmengen von 0,1 - 10 Gew.-%, bevorzugt 0,5 - 5,0 Gew.-% und besonders bevorzugt 1,0 - 4,0 Gew.-%, bezogen auf die gesamte erfindungsgemäße Zusammensetzung, enthalten.

Weitere erfindungsgemäß besonders bevorzugte Zusammensetzungen sind dadurch gekennzeichnet, dass sie neben dem mindestens einen erfindungsgemäß verwendeten Wirkstoff, ausgewählt aus Hydroxystilben-Oligomeren, Alkylethern hiervon und Estern hiervon, das Enzym Photolyase in einer Gesamtmenge von 0,0001 - 0,1 Gew.-%, bevorzugt 0,001 - 0,05 Gew.-%, besonders bevorzugt 0,002 - 0,02 Gew.-% und außerordentlich bevorzugt 0,005 - 0,01 Gew.-%, jeweils bezogen auf die gesamte erfindungsgemäße Zusammensetzung, enthalten.

Weitere erfindungsgemäß besonders bevorzugte Zusammensetzungen sind dadurch gekennzeichnet, dass sie neben dem mindestens einen erfindungsgemäß verwendeten Wirkstoff, ausgewählt aus Hydroxystilben-Oligomeren, Alkylethern hiervon und Estern hiervon, das Enzym T4 Endonuclease V in einer Gesamtmenge von 0,0001 - 0,1 Gew.-%, bevorzugt 0,001 - 0,05 Gew.-%, besonders bevorzugt 0,002 - 0,02 Gew.-% und außerordentlich bevorzugt 0,005 - 0,01 Gew.-%, jeweils bezogen auf die gesamte erfindungsgemäße Zusammensetzung, enthalten.

Erfindungsgemäß besonders bevorzugte Zusammensetzungen sind gekennzeichnet durch einen Gehalt an trans-epsilon-Viniferin und Photolyase.

Weitere erfindungsgemäß besonders bevorzugte Zusammensetzungen sind gekennzeichnet durch einen Gehalt an Vitisin E und Photolyase.

Weitere erfindungsgemäß besonders bevorzugte Zusammensetzungen sind gekennzeichnet durch einen Gehalt an Vitisin D und Photolyase.

Weitere erfindungsgemäß besonders bevorzugte Zusammensetzungen sind gekennzeichnet durch einen Gehalt an trans-epsilon-Viniferin und T4 Endonuclease V.

Weitere erfindungsgemäß besonders bevorzugte Zusammensetzungen sind gekennzeichnet durch einen Gehalt an Vitisin E und T4 Endonuclease V.

Weitere erfindungsgemäß besonders bevorzugte Zusammensetzungen sind gekennzeichnet durch einen Gehalt an Vitisin D und T4 Endonuclease V.

Weitere erfindungsgemäß besonders bevorzugte Zusammensetzungen sind gekennzeichnet durch einen Gehalt an trans-epsilon-Viniferin, Photolyase und T4 Endonuclease V.

Weitere erfindungsgemäß besonders bevorzugte Zusammensetzungen sind gekennzeichnet durch einen Gehalt an Vitisin E, Photolyase und T4 Endonuclease V.

Weitere erfindungsgemäß besonders bevorzugte Zusammensetzungen sind gekennzeichnet durch einen Gehalt an Vitisin D, Photolyase und T4 Endonuclease V.

Es ist weiterhin bevorzugt, dass in den vorstehend genannten Zusammensetzungen ein Alkylether des (E)-Hydroxystilben-Oligomers mit Photolyase und/oder T4 Endonuclease V kombiniert wird.

Es ist weiterhin bevorzugt, dass in den vorstehend genannten Zusammensetzungen ein Ester des (E)-Hydroxystilben-Oligomers mit Photolyase und/oder T4 Endonuclease V kombiniert wird.

Erfindungsgemäß besonders bevorzugte Zusammensetzungen sind dadurch gekennzeichnet, dass sie mindestens ein Sojaproteinhydrolysat mit einem mittleren Molekulargewicht (Mw) im Bereich von 1200 - 1800 Dalton, bevorzugt im Bereich von 1400 - 1700 Dalton, in einer Gesamtmenge von 0,0001 - 5 Gew.-%, bevorzugt 0,001 - 1 Gew.-%, besonders bevorzugt 0,01 - 0,1 Gew.-% und außerordentlich bevorzugt 0,02 - 0,05 Gew.-%, enthalten, jeweils bezogen auf den Gehalt an Aktivsubstanz in der gesamten Zusammensetzung.

Erfindungsgemäß besonders bevorzugte Zusammensetzungen sind dadurch gekennzeichnet, dass sie mindestens ein Sojaproteinhydrolysat mit einem mittleren Molekulargewicht (Mw) im Bereich von 600 - 1000 Dalton, bevorzugt 800 Dalton, in einer Gesamtmenge von 0,0001 - 1 Gew.-%, bevorzugt 0,001 - 0,1 Gew.-%, besonders bevorzugt 0,005 - 0,05 Gew.-% und außerordentlich bevorzugt 0,006 - 0,01 Gew.-%, enthalten, jeweils bezogen auf den Gehalt an Aktivsubstanz in der gesamten Zusammensetzung.

Erfindungsgemäß besonders bevorzugte Zusammensetzungen sind dadurch gekennzeichnet, dass sie mindestens ein Weizenproteinhydrolysat in einer Gesamtmenge von 0,001 - 5,0 Gew.-%, bevorzugt 0,01 - 1,0 Gew.-%, besonders bevorzugt 0,1 - 0,5 Gew.-% und außerordentlich bevorzugt 0,2 - 0,4 Gew.-%, enthalten, jeweils bezogen auf den Gehalt an Aktivsubstanz in der gesamten Zusammensetzung.

Erfindungsgemäß besonders bevorzugte kosmetische oder dermatologische Zusammensetzungen sind dadurch gekennzeichnet, dass sie zusätzlich zu dem mindestens einen erfindungsgemäß verwendeten Wirkstoff, ausgewählt aus Hydroxystilben-Oligomeren, Alkylethern hiervon und Estern hiervon, a) mindestens ein Sojaproteinhydrolysat mit einem mittleren Molekulargewicht im Bereich von 1200 - 1800 Dalton, bevorzugt im Bereich von 1400 - 1700 Dalton b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat in Gewichtsverhältnissen von 1 : (0,1 - 1 ) : (1 - 50), bevorzugt 1 : (0,2 - 0,6) : (10 - 20), enthalten.

Erfindungsgemäß besonders bevorzugte Zusammensetzungen sind dadurch gekennzeichnet, dass sie neben dem mindestens einen erfindungsgemäß verwendeten Wirkstoff, ausgewählt aus Hydroxystilben-Oligomeren, Alkylethern hiervon und Estern hiervon, mindestens einen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, in einer Gesamtmenge von 0,00001 - 2 Gew.-%, bevorzugt 0,0001 - 1 Gew.-%, besonders bevorzugt 0,001 - 0,1 Gew.-% und außerordentlich bevorzugt 0,01 - 0,05 Gew.-%, jeweils bezogen auf den Gehalt an Aktivsubstanz in der gesamten Zusammensetzung, enthalten. Erfindungsgemäß besonders bevorzugte Zusammensetzungen sind dadurch gekennzeichnet, dass sie zusätzlich zu trans-epsilon-Viniferin a) mindestens ein Sojaproteinhydrolysat mit einem mittleren Molekulargewicht im Bereich von 1200 - 1800 Dalton, bevorzugt im Bereich von 1400 - 1700 Dalton b) mindestens ein Sojaproteinhydrolysat mit einem mittleren Molekulargewicht im Bereich von 600 - 1000 Dalton, bevorzugt 800 Dalton, und c) mindestens ein Weizenproteinhydrolysat in Gewichtsverhältnissen von 1 : (0,1 - 1) : (1 - 50), bevorzugt 1 : (0,2 - 0,6) : (10 - 20), enthalten.

Erfindungsgemäß besonders bevorzugte Zusammensetzungen sind dadurch gekennzeichnet, dass sie zusätzlich zu trans-epsilon-Viniferin mindestens einen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, in einer Gesamtmenge von 0,00001 - 2 Gew.-%, bevorzugt 0,0001 - 1 Gew.-%, besonders bevorzugt 0,001 - 0,1 Gew.-% und außerordentlich bevorzugt 0,01 - 0,05. Gew.-%, jeweils bezogen auf den Gehalt an Aktivsubstanz in der gesamten Zusammensetzung, enthalten.

Erfindungsgemäß besonders bevorzugte Zusammensetzungen sind gekennzeichnet durch einen Gehalt an trans-epsilon-Viniferin und und N-Palmitoyl-Gly-His-Lys.

Weitere erfindungsgemäß besonders bevorzugte Zusammensetzungen sind gekennzeichnet durch einen Gehalt an Vitisin E und und N-Palmitoyl-Gly-His-Lys.

Weitere erfindungsgemäß besonders bevorzugte Zusammensetzungen sind gekennzeichnet durch einen Gehalt an Vitisin D und und N-Palmitoyl-Gly-His-Lys.

Weitere erfindungsgemäß besonders bevorzugte Zusammensetzungen sind gekennzeichnet durch einen Gehalt an trans-epsilon-Viniferin und N-Palmitoyl-Gly-Gln-Pro-Arg.

Weitere erfindungsgemäß besonders bevorzugte Zusammensetzungen sind gekennzeichnet durch einen Gehalt an Vitisin E und N-Palmitoyl-Gly-Gln-Pro-Arg.

Weitere erfindungsgemäß besonders bevorzugte Zusammensetzungen sind gekennzeichnet durch einen Gehalt an Vitisin D und N-Palmitoyl-Gly-Gln-Pro-Arg.

Weitere erfindungsgemäß besonders bevorzugte Zusammensetzungen sind gekennzeichnet durch einen Gehalt an trans-epsilon-Viniferin, und N-Palmitoyl-Gly-His-Lys und N-Palmitoyl-Gly-Gln-Pro-Arg.

Weitere erfindungsgemäß besonders bevorzugte Zusammensetzungen sind gekennzeichnet durch einen Gehalt an Vitisin E, und N-Palmitoyl-Gly-His-Lys und N-Palmitoyl-Gly-Gln-Pro-Arg. Weitere erfindungsgemäß besonders bevorzugte Zusammensetzungen sind gekennzeichnet durch einen Gehalt an Vitisin D, und N-Palmitoyl-Gly-His-Lys und N-Palmitoyl-Gly-Gln-Pro-Arg. Es ist weiterhin bevorzugt, dass in den vorstehend genannten Zusammensetzungen ein Alkylether des (E)-Hydroxystilben-Oligomers mit N-Palmitoyl-Gly-His-Lys und/oder N-Palmitoyl-Gly-Gln-Pro-Arg kombiniert wird.

Es ist weiterhin bevorzugt, dass in den vorstehend genannten Zusammensetzungen ein Ester des (E)-Hydroxystilben-Oligomers mit N-Palmitoyl-Gly-His-Lys und/oder N-Palmitoyl-Gly-Gln-Pro-Arg kombiniert wird.

Erfindungsgemäß besonders bevorzugte Zusammensetzungen sind gekennzeichnet durch einen Gehalt an trans-epsilon-Viniferin und und N-Palmitoyl-Lys-Thr-Thr-Lys-Ser.

Weitere erfindungsgemäß besonders bevorzugte Zusammensetzungen sind gekennzeichnet durch einen Gehalt an Vitisin E und und N-Palmitoyl-Lys-Thr-Thr-Lys-Ser.

Weitere erfindungsgemäß besonders bevorzugte Zusammensetzungen sind gekennzeichnet durch einen Gehalt an Vitisin D und und N-Palmitoyl-Lys-Thr-Thr-Lys-Ser.

Es ist weiterhin bevorzugt, dass in den vorstehend genannten Zusammensetzungen ein Alkylether des (E)-Hydroxystilben-Oligomers mit N-Palmitoyl-Lys-Thr-Thr-Lys-Ser kombiniert wird.

Es ist weiterhin bevorzugt, dass in den vorstehend genannten Zusammensetzungen ein Ester des (E)-Hydroxystilben-Oligomers mit N-Palmitoyl-Lys-Thr-Thr-Lys-Ser kombiniert wird.

Es kann erfindungsgemäß besonders bevorzugt sein, dass die als Wirkstoff eingesetzten Aminosäuren und Peptide zur Verbesserung der Penetration in die Haut mit mindestens einer, bevorzugt linearen, C₂-C₂₂-Fettsäure N-acyliert und/oder verestert sind. Zur N-Acylierung und/oder Veresterung besonders bevorzugt sind C₈-C₁₈-Fettsäuren, ganz besonders bevorzugt sind Myristinsäure (C₁₄) und Palmitinsäure (C₁₆). Besonders bevorzugt ist weiterhin, dass alle verwendeten Aminosäuren und Peptide derartig N-acyliert und/oder verestert sind. Ebenfalls bevorzugt ist die Substitution der Aminosäuren und Peptide mit einer Benzyloxycarbonylgruppe an der terminalen Aminogruppe.

In einer weiteren bevorzugten Ausführungsform der Erfindung liegen die Monomeren und Oligomeren von Aminosäuren, N-C₂-C₂₄-Acylaminosäuren, den Estern und/oder den physiologisch verträglichen Metallsalzen dieser Substanzen in geträgerter Form vor, insbesondere aufgetragen auf feinteiligen, pulverförmigen Substraten wie Kieselgel, insbesondere Aerosil-Typen, weiterhin Talkum, Microsponges, modifizierten Stärken und Stärkederivaten, kristalliner Cellulose, Cellulosepulvern, Lactoglobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

Ein weiterer erfindungsgemäß bevorzugter Wirkstoff, der in Gegenwart von dem mindestens einen erfindungsgemäß verwendeten Wirkstoff, ausgewählt aus Hydroxystilben-Oligomeren, Alkylethern hiervon und Estern hiervon, eine unerwartete Wirkungssteigerung zeigt, ist ausgewählt aus Polysacchariden.

Überraschend wurde festgestellt, dass die Wirkung des erfindungsgemäß verwendeten Hydroxystilben-Oligomer(derivat)s in unerwarteter Weise durch Polysaccharide, insbesondere durch Polysaccharide, die mindestens einen Desoxyzucker-Baustein, insbesondere Fucose und/oder Rhamnose, enthalten, und/oder durch Glycosaminoglycane und/oder durch Glucane gesteigert werden kann. Ohne an diese Theorie gebunden sein zu wollen, wird vermutet, dass der Zusatz von mindestens einem Polysaccharid zu einer optimierten Regulierung des Wasserhaushalts innerhalb der extracellulären Matrix (ECM) und/oder zu einer optimierten Regulierung der Nährstoffversorgung innerhalb der ECM und/oder zu einer Stimulierung der Aufbauprozesse der ECM führt, wodurch die Wirkung der erfindungsgemäß verwendeten Hydroxystilben-Oligomere synergistisch unterstützt werden kann und wodurch der Übergang der Zellen, insbesondere der Zellen der Epidermis und/oder der Dermis aus dem proliferativen Status in den ruhenden, seneszenten Status noch stärker verzögert, und/oder der Anteil der Zellen, insbesondere der Zellen in der Epidermis und/oder der Dermis, die sich im ruhenden, seneszenten Status befinden, weiter verringert werden kann.

Erfindungsgemäß bevorzugte Polysaccharide sind solche, die mindestens einen Desoxyzucker-Baustein, insbesondere Fucose und/oder Rhamnose, enthalten, besonders bevorzugt ausgewählt aus Substanzen mit den INCI-Bezeichnungen Biosaccharide Gum-1 (z.B. in dem Handelsprodukt Fucogel^{®} von Solabia), Biosaccharide Gum-2 (z. B. in dem Handelsprodukt Rhamnosoft^{®} von Solabia), Biosaccharide Gum-3 (z. B. in dem Handelsprodukt Fucogenol^{®} von Solabia) und Biosaccharide Gum-4 (z. B. in dem Handelsprodukt Glycofilm^{®} von Solabia), weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich z. B. als Handelsprodukt Elastinol plus^{®} von Solabia. Weitere erfindungsgemäß bevorzugte Polysaccharide sind ausgewählt aus den Glycosaminoglycanen, besonders bevorzugt Hyaluronsäure und deren Salze, Dextran, Dextransulfat, Chondroitin-4-sulfat und Chondroitin-6-sulfat.

Weitere erfindungsgemäß bevorzugte Polysaccharide sind ausgewählt aus den Glucanen. Bei Glucanen oder Polyglucosanen handelt es sich um eine Klasse von Homopolysacchariden, deren Monomerbaustein ausschließlich Glucose ist. Das Glucose-Molekül kann α-glycosidisch oder β-glycosidisch verknüpft, unterschiedlich stark verzweigt oder linear angeordnet sein.

Beispiele für Glucane sind Cellulose, Amylose, Glycogen, Lichenin, Laminarin aus Algen, Pachyman aus Baumpilzen und Hefeglucane mit β-1,3-Bindung; Nigeran, ein aus Pilzen isoliertes Mycodextran (a-1,3-Glucan, a-1,4-Glucan) und Pustulan (β-1,6-Glucan).

Glucane sind unter anderem Bestandteil der Zellwand; sie werden von zahlreichen Mikroorganismen unter bestimmten physiologischen Bedingungen in ihr Milieu abgegeben oder auch erst dort synthetisiert. Dextran, ein primär α-1,6-gebundenes D-Glucan, ist das bedeutendste in dieser Polysaccharid-Gruppe. Weitere technisch interessante und erfindungsgemäß hervorragend geeignete Glucane sind Curdlan (β-1,3-D-Glucan), Pullulan (α-1,4-gebundenes und α-1,6-gebundenes D-Glucan) und Schizophyllan (β-1,3-Grundkette, β-1,6-Seitenkette). Glucane besitzen diverse immunmodulatorische Eigenschaften, auf Zelloberflächen des menschlichen Immunsystems befinden sich entsprechende Glucan-Rezeptoren.

Weitere erfindungsgemäß bevorzugte Wirkstoffe, die in Gegenwart von dem mindestens einen erfindungsgemäß verwendeten Wirkstoff, ausgewählt aus Hydroxystilben-Oligomeren, Alkylethern hiervon und Estern hiervon, eine unerwartete Wirkungssteigerung zeigen, sind ausgewählt aus beta-(1,3-1,4)-Glucanen. Besonders bevorzugt sind beta-(1,3-1,4)-Glucane, die zu etwa 70% 1,4-Verknüpfungen und zu etwa 30% 1,3-Verknüpfungen aufweisen. Derartige beta-(1,3-1,4)-Glucane sind bevorzugt erhältlich aus Extrakten aus Haferkörnern (Avena Sativa (Oat) Kernel Extract). Ein erfindungsgemäß besonders bevorzugter Extrakt aus Haferkörnern ist unter der Handelsbezeichnung Drago Beta Glucan (02/060800) von der Firma Symrise erhältlich.

Weitere erfindungsgemäß bevorzugte Polysaccharide, die in Gegenwart von dem mindestens einen erfindungsgemäß verwendeten Wirkstoff, ausgewählt aus Hydroxystilben-Oligomeren, Alkylethern hiervon und Estern hiervon, eine unerwartete Wirkungssteigerung zeigen, sind ausgewählt aus chemisch modifizierten Cellulose-Derivaten, vorzugsweise Hydroxyalkylcellulosen, wie Hydroxyethylcellulose, Hydroxypropylcellulose, Hydroxypropylmethylcellulose, Methylcellulose, Hydroxymethylcellulose, Carboxymethylcellulose, weiterhin insbesondere aus Stärken sowie Stärkeabbauprodukten wie Amylose und Amylopektin, chemisch und/oder thermisch modifizierte Stärken, z. B. Hydroxypropylstärkephosphat, Dihydroxypropyldistärkephosphat, Aluminiumstärkeoctenylsuccinat, Natriumstärkeoctenylsuccinat oder Calciumstärkeoctenylsuccinat, weiterhin Chitosan und dessen Salzen und anderen Derivaten, weiterhin aus Fucose- und Rhamnosefreien Polysacchariden, die Gums oder Gummen bilden, wie beispielsweise Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar.

Erfindungsgemäß besonders bevorzugte Zusammensetzungen sind dadurch gekennzeichnet, dass sie neben dem mindestens einen erfindungsgemäß verwendeten Wirkstoff, ausgewählt aus Hydroxystilben-Oligomeren, Alkylethern hiervon und Estern hiervon, mindestens ein Polysaccharid in einer Gesamtmenge von 0,001 - 10 Gew.-%, bevorzugt 0,01 - 5 Gew.-%, besonders bevorzugt 0,1-3 Gew.-% und außerordentlich bevorzugt 0,5 - 1 Gew.-%, jeweils bezogen auf den Gehalt an Aktivsubstanz Polysaccharid in der gesamten Zusammensetzung, enthalten. Erfindungsgemäß besonders bevorzugte Zusammensetzungen sind gekennzeichnet durch einen Gehalt an trans-epsilon-Viniferin und Hyaluronsäure(salz).

Weitere erfindungsgemäß besonders bevorzugte Zusammensetzungen sind gekennzeichnet durch einen Gehalt an Vitisin E und Hyaluronsäure(salz).

Weitere erfindungsgemäß besonders bevorzugte Zusammensetzungen sind gekennzeichnet durch einen Gehalt an Vitisin D und Hyaluronsäure(salz).

Weitere erfindungsgemäß besonders bevorzugte Zusammensetzungen sind gekennzeichnet durch einen Gehalt an trans-epsilon-Viniferin und beta-Glucan.

Weitere erfindungsgemäß besonders bevorzugte Zusammensetzungen sind gekennzeichnet durch einen Gehalt an Vitisin E und beta-Glucan.

Weitere erfindungsgemäß besonders bevorzugte Zusammensetzungen sind gekennzeichnet durch einen Gehalt an Vitisin D und beta-Glucan.

Weitere erfindungsgemäß besonders bevorzugte Zusammensetzungen sind gekennzeichnet durch einen Gehalt an trans-epsilon-Viniferin und Natriumstärkeoctenylsuccinat.

Weitere erfindungsgemäß besonders bevorzugte Zusammensetzungen sind gekennzeichnet durch einen Gehalt an Vitisin E und Natriumstärkeoctenylsuccinat.

Weitere erfindungsgemäß besonders bevorzugte Zusammensetzungen sind gekennzeichnet durch einen Gehalt an Vitisin D und Natriumstärkeoctenylsuccinat.

Es ist weiterhin bevorzugt, dass in den vorstehend genannten Zusammensetzungen ein Alkylether des (E)-Hydroxystilben-Oligomers mit Hyaluronsäure(salz), beta-Glucan und/oder Natriumstärkeoctenylsuccinat kombiniert wird.

Es ist weiterhin bevorzugt, dass in den vorstehend genannten Zusammensetzungen ein Ester des (E)-Hydroxystilben-Oligomers mit Hyaluronsäure(salz), beta-Glucan und/oder Natriumstärkeoctenylsuccinat kombiniert wird.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen weiteren, die Kollagensynthese stimulierenden Wirkstoff enthalten, der ausgewählt ist aus
- Retinoiden, insbesondere Retinol und C₂-C₂₂-Fettsäureestern des Retinols,
- Kombucha,
- Olivenblattextrakten (Olea Europaea (Olive) Leaf Extract),
- Oleanolsäure,
- Oleanol,
- Grüntee-Extrakten (Camellia Sinensis),
- Kreatin,
- sowie Mischungen der vorgenannten Substanzen.

Weitere erfindungsgemäß bevorzugte Wirkstoffe, die die Kollagensynthese stimulieren, sind ausgewählt aus Retinoiden. Zu den Retinoiden werden im Sinne der vorliegenden Erfindung Retinol (Vitamin A₁), die C₂-C₂₂-Fettsäureester des Retinols (= Retinylester, insbesondere Retinylpalmitat und Retinylacetat), 3,4-Didehydroretinol (Vitamin A₂), Retinal und seine Isomere, *all-trans*-Retinsäure, 9-*cis*-Retinsäure und 13-cis-Retinsäure (Tretinoin), die Ester der Retinsäure sowie weitere verwandte Substanzen (synthetische Retinoide) gezählt, die für ihre vielfältige biologische Wirkung insbesondere auf Wachstum und Differenzierung bekannt sind, sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen. Synthetische Retinoide werden in drei Gruppen eingeteilt: nichtaromatisch (z. B. *Isotretinoin*), monoaromatisch (z.B. Acitretin) und polyaromatisch (sogenannte Arotinoide, z. B. Tazarotene, die eine spezifische Wirkung auf einzelne Retinoid-Rezeptoren haben). Erfindungsgemäß besonders bevorzugte Retinoide sind Retinol und die C₂-C₂₂-Fettsäureester des Retinols, insbesondere Retinylpalmitat und Retinylacetat.

Weitere erfindungsgemäß bevorzugte Wirkstoffe, die die Kollagensynthese stimulieren, sind ausgewählt aus Kombucha.

Bei Kombucha handelt es sich um ein aus dem ostasiatischen Raum stammendes, etwa 0,5 Vol.-% Alkohol enthaltendes, angenehm säuerliches, leicht süßes, moussierendes Gärungsgetränk auf Basis von Mono- oder Disaccharid-haltigem Tee (Schwarzer Tee, Grüner Tee, Kräutertee oder Früchtetee), das ohne Zusatz von Konservierungsstoffen, Farbstoffen und Aromastoffen hergestellt wird. Beim "Kombucha-Teepilz" handelt es sich tatsächlich nicht um einen Pilz, sondern um eine Symbiose von Essigsäurebakterien (meistens *Acetobacter xylinum,* aber auch *Acetobacter gluconicum*) mit verschiedenen Hefen *(Saccharomyces* sp., *Torula* sp., *Pichia fermentans*); fakultativ können Milchsäurebakterien vorhanden sein. Aus Gründen der Produktionssicherheit und Produktionsstabilität wird industriell gefertigter Kombucha oft wärmebehandelt.

Unter "Tee" werden erfindungsgemäß wässrige Aufgüsse von Blättern, Knospen und zarten Stielen von Thea sinensis (Camellia sinensis) und Thea assamica, aber auch von diversen anderen Pflanzen verstanden, beispielsweise Minze, Linde (Lindenblüten), Malve, Rotbusch, Kamille usw.

Bevorzugte Kombucha ist aus Mono- oder Disaccharid-haltigem Tee von fermentierten (Schwarztee, Oolong-Tee, Gelber Tee, Pu-Erh-Tee) oder unfermentierten (Grüntee) Blättern von Thea sinensis (Camellia sinensis) oder Thea assamica gewonnen. Weitere bevorzugte Kombucha ist durch Fermentation von (meist mit Saccharose) gezuckertem Tee mit den symbiotischen Mikroorganismen Saccharomyces und Acetobacter xylinum gewonnen. Ein erfindungsgemäß besonders bevorzugtes Kombucha-Produkt ist ein Produkt, das durch Fermentation von mit Saccharose gezuckertem Schwarztee mit den zwei symbiotischen Mikroorganismen Saccharomyces und Acetobacter xylinum gewonnen wurde und beispielsweise unter der INCI-Bezeichnung "Saccharomyces/Xylinum/Black Tea Ferment, Glycerin, Hydroxyethylcellulose" mit der Handelsbezeichnung "Kombuchka" als Glycerin-haltige verdickte Zubereitung von der Firma Sederma erhältlich ist.

Weitere erfindungsgemäß bevorzugte Wirkstoffe, die die Kollagensynthese stimulieren, sind ausgewählt aus Olivenblattextrakten (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.

Olivenblattextrakte können einen hohen Gehalt an Oleanolsäure und/oder Oleanol aufweisen. Weitere erfindungsgemäß bevorzugte Wirkstoffe, die die Kollagensynthese stimulieren, sind ausgewählt aus Oleanolsäure und Oleanol.

Weitere erfindungsgemäß bevorzugte Wirkstoffe, die die Kollagensynthese stimulieren, sind ausgewählt aus Grüntee-Extrakten (Camellia Sinensis). Ein erfindungsgemäß besonders bevorzugter Grüntee-Extrakt ist liposomenverkapselter Grüntee-Extrakt, beispielsweise unter der Handelsbezeichnung Greenselect Phytosome von der Firma Indena erhältlich.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen weiteren, die Kollagensynthese stimulierenden Wirkstoff in einer Gesamtmenge von 0,000001 - 5 Gew.-%, bevorzugt 0,00001 - 2 Gew.-%, besonders bevorzugt 0,0001 - 1 Gew.-% und außerordentlich bevorzugt 0,005 - 0,5 Gew.-%, enthalten, jeweils bezogen auf den Gehalt an Aktivsubstanz in der gesamten Zusammensetzung.

Eine maßgebliche Folge der Hautalterung ist der Verlust an Kollagen. Dieser basiert zum einen auf einer verminderten Kollagensynthese in bestimmten Hautzellen, den Fibroblasten, zum anderen auf einem verstärkten Kollagenabbau, insbesondere durch bestimmte Enzyme, wie der sogenannten Matrix-Metalloproteinase-1 (MMP-1). Insbesondere führt die UV-induzierte Expression der Matrix-Metalloproteinase-1 zu einem Abbau von Kollagen-1, das ca. 85% des totalen Proteins in der extrazellulären Matrix ausmacht. Neben den Elementen des Zytoskeletts und den Gap Junction-Proteinen vermitteln weitere Proteine dem Hautgewebe mechanische Stabilität. Diese Proteine vermitteln eine Bindung zwischen Zellen und extrazellulärer Matrix (ECM). Bestandteil der ECM ist z. B. das Kollagen oder auch die Hyaluronsäure. Für die Bindung von Zellen an die Proteine der ECM sind Rezeptoren verantwortlich, die auf der Zelloberfläche lokalisiert sind. So bindet z.B. der Oberflächenrezeptor CD44, dessen Expression beispielsweise durch Apfelkernextrakte gesteigert wird, an Hyaluronsäure.

Neuere Untersuchungen haben die Annahme erhärtet, dass durch den Kollagenabbau das Integrin-vermittelte Wechselspiel zwischen Kollagenmatrix und Fibroblasten gestört wird. Es wurde gezeigt, dass auf einer vorgeschädigten, kontrahierten und spannungsarmen Kollagenmatrix die weitere Kollagensynthese nur eingeschränkt erfolgt. Der Verlust an mechanischer Spannung scheint kausal an der verminderten Neusynthese von Pro-Kollagen beteiligt zu sein (J. Varani et al., Reduced Fibroblast Interaction with Intact Collagen as a mechanism for depressed Collagen Synthesis in Photodamaged Skin, J. Invest. Dermatol. 122, 1471 - 1479, 2004).

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen weiteren, die Interaktion zwischen der extrazellulären Matrix und den Fibroblasten erhöhenden und/oder verbessernden Wirkstoff enthalten, der ausgewählt ist aus
- Apfelkernextrakten (Pyrus Malus (Apple) Fruit Extract),
- Lotuskeim-Extrakten (Nelumbo Nucifera Germ Extract),
- Extrakten aus Maiskörnern (Zea Mays (Corn) Kernel Extract),
- Extrakten aus Schwarzen Holunderblüten (Sambucus Nigra Flower Extract),
- Mischungen aus mindestens einem Extrakt aus Kakaobohnen (Theobroma cacao) und mindestens einem Extrakt aus den Blättern der Pfefferminze (Mentha piperita),
- Resveratrol und/oder Resveratrolmono-, -di- und -triphosphorsäureestern und deren Salzen und/oder Resveratrolmono-, -di- und -trimethylether,
- Isoflavonoiden und Isoflavonoid-reichen Pflanzenextrakten,
- Dihydroquercetin (= Taxifolin),
- sowie Mischungen hiervon.

Weitere erfindungsgemäß bevorzugte Wirkstoffe, die die Interaktion zwischen der extrazellulären Matrix und den Fibroblasten erhöhen und/oder verbessern, sind ausgewählt aus Apfelkernextrakten (Pyrus Malus (Apple) Fruit Extract). Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCl: PEG-40 Hydrogenated Castor Oil, PPG-2-Ceteareth-9, Pyrus Malus (Apple) Fruit Extract), zum anderen in fettlöslicher Form als Ederline-L (INCl: Hexyldecanol, Pyrus Malus (Apple) Fruit Extract) erhältlich.

Erfindungsgemäß besonders bevorzugte kosmetische oder dermatologische Zusammensetzungen sind dadurch gekennzeichnet, dass sie den Rohstoff Ederline in Mengen von 0,1 - 10 Gew.-%, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Apfelkernextrakt in Mengen von 0,001 - 2 Gew.-%, bevorzugt 0,01 - 1,6 Gew.-% und besonders bevorzugt 0,03 - 1 Gew.-%, jeweils bezogen auf den Gehalt an Aktivsubstanz in der gesamten Zusammensetzung, enthalten.

Weitere erfindungsgemäß bevorzugte Wirkstoffe, die die Interaktion zwischen der extrazellulären Matrix und den Fibroblasten erhöhen und/oder verbessern, sind ausgewählt aus Lotuskeim-Extrakten. Ein erfindungsgemäß besonders bevorzugter Lotuskeim-Extrakt ist unter der Handelsbezeichnung Lotus Germ Extract mit der INCI-Bezeichnung Water, Butylene Glycol, Nelumbo Nucifera Germ Extract von der Firma Maruzen erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Schwarzen Holunderblüten (Sambucus Nigra Flower Extract). Ein erfindungsgemäß besonders bevorzugter Extrakt aus Schwarzen Holunderblüten ist unter der Handelsbezeichnung Sambucus AO von der Firma Alpaflor/Centerchem bzw. von Permcos erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Mischungen aus mindestens einem Extrakt aus Kakaobohnen (Theobroma cacao) und mindestens einem Extrakt aus den Blättern der Pfefferminze (Mentha piperita). Erfindungsgemäß besonders bevorzugte Mischungen aus mindestens einem Extrakt aus Kakaobohnen (Theobroma cacao) und mindestens einem Extrakt aus den Blättern der Pfefferminze (Mentha piperita) - wobei wässrige, glycolische oder wässrig-glycolische Zubereitungen dieser Extraktmischungen besonders bevorzugt sind - sind unter den Handelsnamen Caomint, Caophenol, Caobromine, Caospice und Caoorange von Solabia erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Resveratrol (trans-Stilben-3,4'-5-triol) und/oder Resveratrolmono-, -di- und -triphosphorsäureestern und deren Salzen und/oder Resveratrolmono-, -di- und -trimethylether. Ein erfindungsgemäß besonders bevorzugter Resveratrolphosphorsäureester ist Trisodium Resveratrol Triphosphate, z. B. erhältlich von Ajinomoto.

Weitere erfindungsgemäß bevorzugte Wirkstoffe, die die Interaktion zwischen der extrazellulären Matrix und den Fibroblasten erhöhen und/oder verbessern, sind ausgewählt aus Isoflavonoiden und Isoflavonoid-reichen Pflanzenextrakten.

Zu den Isoflavonoiden zählen erfindungsgemäß die Isoflavone und die Isoflavon-Glycoside.

Unter Isoflavonen sind erfindungsgemäß Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Besonders bevorzugte Isoflavone sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Weiterhin ist es erfindungsgemäß bevorzugt, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Lipobelle Soyaglycone (Mibelle AG Cosmetics), Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.

Erfindungsgemäß besonders bevorzugte Zusammensetzungen sind dadurch gekennzeichnet, dass sie mindestens ein Isoflavonoid in Gesamtmengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

Ein weiterer erfindungsgemäß bevorzugter Wirkstoff, der die Interaktion zwischen der extrazellulären Matrix und den Fibroblasten erhöht und/oder verbessert, ist Dihydroquercetin (3,3',4',5,7-Pentahydroxyflavanon), das auch als Taxifolin bezeichnet wird.

Erfindungsgemäß besonders bevorzugte Zusammensetzungen sind dadurch gekennzeichnet, dass sie mindestens einen Wirkstoff, der die Interaktion zwischen der extrazellulären Matrix und den Fibroblasten erhöht und/oder verbessert, in einer Gesamtmenge von 0,000001 - 10 Gew.-%, bevorzugt 0,00001 - 5 Gew.-%, besonders bevorzugt 0,0001 - 2 Gew.-% und außerordentlich bevorzugt 0,005 - 0,5 oder 1 Gew.-%, enthalten, jeweils bezogen auf den Gehalt an Aktivsubstanz in der gesamten Zusammensetzung.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen weiteren, die erfindungsgemäße Lehre unterstützenden Wirkstoff enthalten, der ausgewählt ist aus
- DNA- oder RNA-Oligonucleotiden,
- Vitaminen, Provitaminen und Vitaminvorstufen der Gruppen B, E und H und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- anorganischen und organischen UV-Filtersubstanzen,
- selbstbräunenden,
- hautberuhigenden und
- sebumregulierenden Wirkstoffen sowie
- Mischungen dieser Wirkstoffe.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen weiteren, die erfindungsgemäße Lehre unterstützenden Wirkstoff enthalten, der ausgewählt ist aus DNA-Oligonucleotiden und RNA-Oligonucleotiden.

Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glycosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).

Ein erfindungsgemäß besonders bevorzugtes Oligonucleotid ist das Thymidin-Dinucleotid.

In den besonders bevorzugten erfindungsgemäßen Zusammensetzungen sind die DNA-Oligonucleotide oder RNA-Oligonucleotide in Mengen von 0,00001 - 5 Gew.-%, bevorzugt 0,0001 - 1,0 Gew.-% und besonders bevorzugt 0,0005 - 0,5 Gew.-%, bezogen auf die gesamte Zusammensetzung, enthalten.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen Wirkstoff enthalten, der ausgewählt ist aus mindestens einem Vitamin, Provitamin oder einer als Vitaminvorstufe bezeichnete Verbindung aus den Vitamingruppen B, E, H und K und den Estern der vorgenannten Substanzen.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (VIT-1) eingesetzt werden.

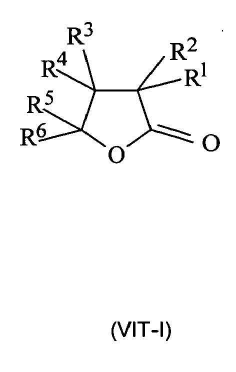

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-y-butyrolacton (Merck), 3,3-Dimethyl-2-hydroxy-y-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.

Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.

Vitamin H ist eine andere Bezeichnung für Biotin oder Vitamin B₇ (siehe oben).

Erfindungsgemäß besonders bevorzugte kosmetische oder dermatologische Zusammensetzungen sind dadurch gekennzeichnet, dass sie mindestens eine der folgenden Wirkstoffkombinationen enthalten:
trans-epsilon-Viniferin und Folsäure,
Vitisin E und Folsäure,
Vitisin D und Folsäure,
trans-epsilon-Viniferin und Folsäure und N-Palmitoyl-Lys-Thr-Thr-Lys-Ser,
trans-epsilon-Viniferin und Carnitin und Folsäure, denn es wurde überraschend festgestellt, dass diese Kombination die Lipolyse in der Subcutis verbessert und dadurch die Festigkeit der Haut verbessert.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen Wirkstoff enthalten, der ausgewählt ist aus mindestens einer α-Hydroxycarbonsäure, α-Ketocarbonsäure oder β-Hydroxycarbonsäure oder deren Ester-, Lacton- und/ oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer- und Mangansalze, die Salze der Alkali-und der Erdalkalimetalle und die Ammonium-, Alkylammonium-, Alkanolammonium- und Glucammoniumsalze, besonders bevorzugt die Natrium-, Kalium-, Magnesium- und Calcium-Salze.

Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.-%, besonders bevorzugt 0,5 - 5 Gew.-% und außerordentlich bevorzugt 1 - 2 Gew.-%, jeweils bezogen auf die gesamte Zusammensetzung, enthalten.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen Wirkstoff enthalten, der ausgewählt ist aus mindestens einem Flavonoid und/oder mindestens einem Flavonoid-reichen Pflanzenextrakt.

Die erfindungsgemäß bevorzugten Flavonoide umfassen die Glycoside der Flavone, der 3-Hydroxyflavone (Flavonole) und der Aurone. Die Flavanone und die Isoflavone sind erfindungsgemäß ausdrücklich von den Flavonoiden ausgenommen. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Rutin (3,3',4',5,7-Pentahydroxyflavon-3-rhamno-glucosid, Quercetin-3-rhamnoglucosid), Troxerutin (3,5-Dihydroxy-3',4',7-tris(2-hydroxyethoxy)-fla-von-3-(6-O-(6-deoxy-a-L-mannopyranosyl)-β-D-glucopyranosid)), Monoxerutin (3,3',4',5-Tetra-hydroxy-7-(2-hydroxyethoxy)flavon-3-(6-O-(6-deoxy-α-L-mannopyranosyl)-β-D-glucopyranosid)), Eriodictin und Apigenin-7-glucosid (4',5,7-Trihydroxyflavon-7-glucosid).

Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.

Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen Wirkstoff enthalten, der ausgewählt ist aus mindestens einem Ubichinon oder einem Ubichinol oder deren Derivaten. Ubichinole sind die reduzierte Form der Ubichinone. Die erfindungsgemäß bevorzugten Ubichinone weisen die Formel (UBI-I) auf: 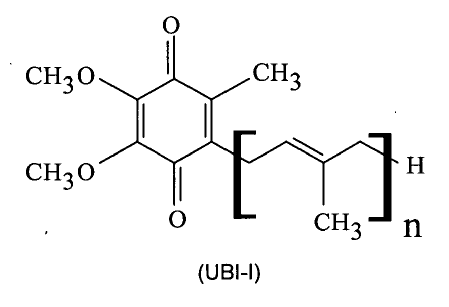 mit n = 6, 7, 8, 9 oder 10.

Besonders bevorzugt ist das Ubichinon der Formel (UBI-I) mit n = 10, auch bekannt als Coenzym Q10.

Erfindungsgemäß bevorzugt werden die Ubichinone, Ubichinole oder deren Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, eingesetzt.

Erfindungsgemäß besonders bevorzugte kosmetische oder dermatologische Zusammensetzungen sind dadurch gekennzeichnet, dass sie mindestens eine der folgenden Wirkstoffkombinationen enthalten: trans-epsilon-Viniferin und Coenzym Q 10, Vitisin E und Coenzym Q 10, Vitisin D und Coenzym Q 10.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen Wirkstoff enthalten, der ausgewählt ist aus Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.

Erfindungsgemäß bevorzugt wird Silymarin in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, eingesetzt.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen Wirkstoff enthalten, der ausgewählt ist aus Ectoin. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

Erfindungsgemäß besonders bevorzugte kosmetische oder dermatologische Zusammensetzungen sind dadurch gekennzeichnet, dass sie mindestens eine der folgenden Wirkstoffkombinationen enthalten: trans-epsilon-Viniferin und Ectoin, Vitisin E und Ectoin, Vitisin D und Ectoin.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens eine anorganische und/oder mindestens eine organische UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.

Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/ Dimethylsiloxan-Copolymer mit der INCl-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCl: Bis-Ethylhexyloxyphenol Methoxyphenyl Triazin, unter dem Namen Tinosorb S bei CIBA erhältlich), Dioctylbutylamidotriazon (INCl: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl) - benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amyl-phenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarb-oxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCl Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethyl-hexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Erfindungsgemäß sind die organischen UV-Filtersubstanzen bevorzugt in Mengen von 0,1 - 30 Gew.-%, besonders bevorzugt 0,5 - 20 Gew.-%, außerordentlich bevorzugt 1,0 - 15 Gew.-% und weiter bevorzugt 3,0 - 10 Gew.-%, jeweils bezogen auf die gesamte Zusammensetzung, enthalten.

Erfindungsgemäß sind die anorganischen UV-Filtersubstanzen bevorzugt in Mengen von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, mindestens einen selbstbräunenden Wirkstoff enthalten. Erfindungsgemäß bevorzugte selbstbräunende Wirkstoffe sind ausgewählt aus Dihydroxyaceton, Erythrulose und 5,6-Dihydroxyindolin.

Erfindungsgemäß bevorzugt sind die selbstbräunenden Wirkstoffe in einer Gesamtmenge von 0,01 - 15 Gew.-%, besonders bevorzugt 0,1 - 10 Gew.-%, weiterhin besonders bevorzugt 1,0 - 5 Gew.-% und außerordentlich bevorzugt 2,0 - 4,0 Gew.-%, jeweils bezogen auf die gesamte Zusammensetzung, enthalten.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, weiterhin mindestens einen hautberuhigenden Wirkstoff enthalten. Erfindungsgemäß bevorzugte hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung ,,Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCl : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Cödif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter dem Handelsnamen Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, Extrakten aus den Samen von Cucurbita pepo (Zucchini), Extrakten aus Mentha piperita, insbesondere aus den Blättern von Mentha piperita, N-acylierten Alkanolaminen, insbesondere N-Palmitoylethanolamin, Hydrolysaten und/oder Extrakten aus der Alge *Enteromor-pha compressa* (Darmtang, eine Grünalge), insbesondere aus dem Vegetationskörper (Thallus) der Alge, kommerziell erhältlich z. B. unter dem Handelsnamen Enteline 2, weiterhin ausgewählt aus dem Dipeptid Tyr-Arg, dessen Estern und dessen N-Acyl-Derivaten, insbesondere N-Acetyl-Tyr-Arg-hexyldecylester (z. B. Calmosensine von Sederma, sowie beliebigen Mischungen dieser Substanzen.

Erfindungsgemäß besonders bevorzugte Extrakte aus den Samen von Cucurbita pepo (Zucchini) sind beispielsweise in den Handelsprodukten Curbilene (ex Indena SpA, INCI: Cucurbita pepo (pumpkin) seed extract), Ocaline (ex Soliance, INCI: Sea Water (and) Water (and) Cucurbita pepo (pumpkin) seed extract), ARP 100 (ex Greentech, INCI: Water, Alcohol, Serenoa Serrulata Fruit Extract, Epilobium Angustifolium Extract, Cucurbita Pepo (Pumpkin) Seed Extract), ARP 100 Huileux (ex Greentech, INCI: Caprylic/Capric Triglyceride, Serenoa Serrulata Fruit Extract, Epilobium Angustifolium Flower/Leaf/Stem Extract, Cucurbita Pepo (Pumpkin) Seed Extract), Cucurbitine (ex Christian Dior Parfums, INCI: Water (and) Cucurbita pepo (pumpkin) seed extract), Herbasol Extract Pumpkin (ex Cosmetochem, INCI: Water (and) Propylene glycol (and) Cucurbita pepo (pumpkin) seed extract) und Pumpkin Extract (ex Draco Natural Products, INCI: Water (and) Cucurbita pepo (pumpkin) seed extract) enthalten.

Erfindungsgemäß besonders bevorzugte Extrakte aus Mentha piperita, insbesondere aus den Blättern von Mentha piperita, sind beispielsweise in den Handelsprodukten Calmiskin OP (ex Silab, INCI: Water (and) Mentha piperita (Peppermint) Leaf Extract) und Caomint (ex Solabia, INCI: Propylene Glycol, Water, Mentha piperita (Peppermint) Leaf Extract, Theobroma Cacao (Cocoa) Extract) enthalten.

Die hautberuhigenden Wirkstoffe sind bevorzugt in Mengen von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, weiterhin mindestens einen sebumregulierenden Wirkstoff enthalten. Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques) und den Wirkstoffmischungen Asebiol^{®} BT 2 (Laboratoires Sérobiologiques, INCl: Aqua, Hydrolyzed Yeast Protein, Pyridoxine, Niacinamide, Glycerin, Panthenol, , Allantoin, Biotin) und Antifettfaktor^{®} COS-218/2-A (Cosmetochem, INCl: Aqua, Cetyl-PCA, PEG-8 Isolauryl Thioether, PCA, Cetyl Alcohol). Die sebumregulierenden Wirkstoffe sind bevorzugt in einer Gesamtmenge von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Weitere erfindungsgemäß bevorzugte Zusammensetzungen sind dadurch gekennzeichnet, dass sie zusätzlich zu der Zweierkombination aus mindestens einem erfindungsgemäß verwendeten Wirkstoff, ausgewählt aus Hydroxystilben-Oligomeren, Alkylethern hiervon und Estern hiervon, und mindestens einem weiteren Wirkstoff, ausgewählt aus Aporphin-Alkaloiden, Purin und Purin-Derivaten, natürlichen Betainverbindungen, Harnstoff und alkyl- oder hydroxyalkylsubstituierten Harnstoffen, Monomeren, Oligomeren und Polymeren von Aminosäuren, N-C₂-C₂₄-Acylamino-säuren und/oder den Estern und/oder den physiologisch verträglichen Salzen dieser Substanzen, Polysacchariden, sowie Mischungen dieser Wirkstoffe, weiterhin mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.

Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆-₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, EiLecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCl-Bezeichnungen Dimethiconol, Amodimethicone oder Trimethylsilylamodimethicone.

Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf das gesamte Hautbehandlungsmittel.

Bevorzugt liegen die erfindungsgemäßen kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonders bevorzugten Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-ÖI-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-düsostearat (Handelsprodukt Lameform^{®} TGI) sowie lineare und verzweigte C₈-C₃₀-Fettsäuren und deren Na-, K-, Ammonium-, Ca-, Mg- und Zn-Salze.

Die erfindungsgemäßen Zusammensetzungen enthalten die Emulgatoren bevorzugt in Mengen von 0,1 bis 25 Gew.-%, besonders bevorzugt 0,5 - 15 Gew.-%, bezogen auf die gesamte Zusammensetzung.

In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹ - O - R², in der R¹ eine primäre lineare Alkyl-, Alkenyl- oder Acylgruppe mit 20 - 30 C-Atomen, bevorzugt ein Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt. Eine besonders bevorzugte Verbindung R¹ - O - R² ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythritmonoerucat. Die Anwesenheit von Emulgatoren der allgemeinen Formel R¹ - O - R² kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher dadurch gekennzeichnet, dass sie in Form einer lamellare Strukturen aufweisenden Öl-in-Wasser-Emulsion vorliegen.

In einer weiteren besonders bevorzugten Ausführungsform liegen die erfindungsgemäßen Zusammensetzungen in Form einer Wasser-in-Öl-Emulsion vor. Derartige Wasser-in-Öl-Emulsionen können so formuliert werden, dass die eine höhere Viskosität aufweisen, so dass sie sorgfältig in die Haut einzumassieren sind. Dies unterstützt die Anticellulite-Wirkung der erfindungsgemäßen Zusammensetzungen. Genau so gut lassen sich aber auch niedrig-viskose Wasser-in-Öl-Emulsionen formulieren, insbesondere auf Basis des polymeren Wasser-in-Öl-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-Öl-Emulsionen formulieren.

Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B. F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Weitere bevorzugte Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

Ein weiterer Gegenstand der vorliegenden Erfindung ist eine Zusammensetzung gemäß einem der Ansprüche 12 - 30, die in einem Sprühspender oder Pumpspender verpackt ist.

Ein weiterer Gegenstand der vorliegenden Erfindung ist eine Zusammensetzung gemäß einem der Ansprüche 12 - 26, die in einer zur oralen Verabreichung geeigneten Form konfektioniert ist. Ein weiterer Gegenstand der vorliegenden Erfindung ist eine Zusammensetzung gemäß einem der Ansprüche 12 - 26, die in einer zur Injektion geeigneten Form konfektioniert ist.

### Experimentelle Untersuchungen

### Versuch 1:

Alle Versuche wurden mit einem wasserbasierten Weinrebenextrakt mit einem Gehalt an trans-epsilon-Viniferin von 1,09 Gew.-%, bezogen auf den Extrakt, durchgeführt.

Die für die Versuche verwendeten Testlösungen enthielten 0,01 Gew.-% dieses Weinrebenextrakts, entsprechend einem Gehalt an trans-epsilon-Viniferin von 0,000109 Gew.-%, bezogen auf die Testlösung.

Primäre humane Fibroblasten wurden in acht Ansätzen in Medium mit 0,01 Gew.-% Weinrebenextrakt (siehe oben) über eine Zeitraum von 48 h kultiviert und anschließend die Vitalität über einen WST-Test gegenüber unbehandelten Kontrollzellen bestimmt. Der Versuch wurde jeweils mit Zellen der Passage 2 ("junge Zellen") und mit Zellen der Passage 6 ("ältere Zellen") durchgeführt. Für die mit dem Weinrebenextrakt behandelten Zellen zeigte sich eine gegenüber den Kontrollzellen erhöhte Vitalität, insbesondere wurden die in vitro gealterten Zellen durch die Behandlung deutlich stimuliert.

### Relative Vitalität Weinrebenextrakt-behandelter Fibroblasten im Vergleich zu Kontrollzellen (%)

| **Relative Vitalität Weinrebenextrakt-behandelter Fibroblasten im Vergleich zu Kontrollzellen (%)** | | |
|---|---|---|
| | Mittelwert | Standard-abweichung |
| Passage 2 | 106 | 8 |
| Passage 6 | 112 | 2 |

### Versuch 2:

Primäre humane Fibroblasten wurden in Duplikaten für 48 h in Medium mit 0,01 Gew.-% Weinrebenextrakt (siehe oben) kultiviert, die Teilung der Zellen anschließend durch 12stündige Inkubation mit Thymidin synchronisiert, um dann die Kultivierung weitere 6 Stunden wirkstoff- und thymidinfrei fortzusetzen. Anschließend wurde mittels Durchflusszytometrie die Größe der Zellpopulationen in den einzelnen Phasen des Zellzyklus bestimmt. Die mit dem Weinrebenextrakt (siehe oben) kultivierten Zellen zeigen einen höheren Anteil an Zellen in der proliferativen S- und G2-Phase als die Kontrollzellen, die Zellen werden offenbar schneller reaktiviert.

### Passage 2

| | Probe | G1 | S | G2 | S+G2 | Mittelwert | Stand.-abw. |
|---|---|---|---|---|---|---|---|
| Kontrolle | A | 84,8 | 5 | 5,3 | 10,3 | | |
| | B | 85,7 | 6,1 | 4,7 | 10,8 | 10,6 | 0,4 |
| Weinreben-extrakt 0,01% | A | 85,8 | 6,9 | 3,7 | 10,6 | | |
| | B | 82,9 | 8,1 | 5,6 | 13,7 | 12,2 | 2,2 |

### Passage 6

| | Probe | G1 | S | G2 | S+G₂ | Mittelwert | Stand.-abw. |
|---|---|---|---|---|---|---|---|
| Kontrolle | A | 93,9 | 2 | 3,7 | 5,7 | | |
| | B | 93,4 | 2,5 | 3,6 | 6,1 | 5,9 | 0,3 |
| Weinreben-extrakt 0,01% | A | 89,7 | 5,6 | 4,3 | 9,9 | | |
| | B | 89 | 6,3 | 3,9 | 10,2 | 10,1 | 0,2 |

### Versuch 3:

Primäre humane Fibroblasten wurden über einen Zeitraum von 48 h in Medium mit 0,01 Gew.-% Weinrebenextrakt (siehe oben) kultiviert, anschließend wurde die β-Galaktosidase-Aktivität (Seneszensmarker) im Vergleich zu Kontrollzellen bestimmt. Der Höhe der β-Galaktosidase-Aktivität wurde durch die Wirkstoffbehandlung reduziert, in der Kultur befindet sich nach der Behandlung ein geringerer Anteil senszenter (gealteter) Zellen, die Kultur ist quasi verjüngt.

| Prozentuale beta-Galaktosidase-Aktivität bezogen auf die Kontrolle | | |
|---|---|---|
| Passage 2 | unbehandelt | Weinrebenextrakt (siehe oben) 0,01 Gew.-% |
| MW | 100,0 | 69,3 |
| St.abw. | 18,4 | 23,2 |

| Passage 6 | unbehandelt | Weinrebenextrakt (siehe oben) 0,01 Gew.-% |
|---|---|---|
| MW | 100,0 | 78,8 |
| St.abw. | 10,8 | 5,9 |

### Ausführungsbeispiele

Die nachfolgenden Formulierungsbeispiele sollen den Gegenstand der Erfindung erläutern, ohne ihn hierauf zu beschränken.

Alle Mengenangaben sind in Gew.-%, bezogen auf die gesamte Zusammensetzung.

### 1. Oel-in-Wasser-Emulsionen

### 1. Hautcremes

Die Zusammensetzungen 1 - 4 sind bevorzugte Ausführungsformen von zellverjüngenden Tagescremes.

| | 1 | 2 | 3 | 4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Baysilone-ÖI M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Weinrebenextrakt, wasserbasiert (1,09 Gew.-% trans-epsilon-Viniferin) | 1,00 | 1,00 | 1,00 | 1,00 |
| Caomint | 1,00 | 0,50 | 2,00 | - |
| Calmosensine | 1,00 | 2,00 | - | 2,00 |
| Symdiol68 | 0,30 | 0,50 | - | - |
| DSH-CN | 5,00 | 5,00 | 5,00 | 5,00 |
| Hydrovance | 4,30 | 2,50 | 8,60 | 10,00 |
| Kombuchka | 3,00 | - | - | 3,00 |
| Glistin | - | 2,00 | - | - |
| Natrulon RC 50 DG | - | - | 1,00 | |
| Phytokine | 2,00 | 1,50 | 2,00 | 2,00 |
| Ridulisse C | 0,50 | 0,50 | 0,50 | 0,50 |
| Liftiline | 2,00 | 3,00 | 2,00 | 3,00 |
| Matrixyl 3000 | - | 2,00 | - | - |
| Perfume | 0,10 | 0,10 | 0,10 | 0,10 |
| Aqua | ad 100 | ad 100 | ad 100 | ad 100 |

### 2. Hautcremes:

Die Zusammensetzungen 1 - 3 sind weitere bevorzugte Ausführungsformen von zellverjüngenden Tagescremes für besonders anspruchsvolle Haut mit einem Gehalt an Antiageing-Wirkstoffen.

| | 1 | 2 | 3 |
|---|---|---|---|
| Cetearyl Isononanoate | 4,00 | 4,00 | 4,00 |
| Mineral oil | 6,00 | 6,00 | 6,00 |
| Cutina CBS | 2,00 | 2,00 | 2,00 |
| Palmitic Acid / Stearic Acid | 1,50 | 1,50 | 1,50 |
| Eumulgin B3 | 1,00 | 1,00 | 1,00 |
| Baysilone-Öl M 350 | 1,00 | 1,00 | 1,00 |
| Tocopheryl Acetate | 0,50 | 0,50 | 0,50 |
| Propylparaben | 0,30 | 0,30 | 0,30 |
| Pemulen TR 1 | 0,27 | 0,27 | 0,27 |
| Glycerin | 5,00 | 3,00 | 3,00 |
| Milchsäure 80 % | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Phytokine | 2,00 | 1,50 | 2,00 |
| Ridulisse C | 0,50 | 0,50 | 0,50 |
| Liftiline | 2,00 | 3,00 | 2,00 |
| Sepigel 305 | 0,50 | 0,50 | 0,50 |
| Weinrebenextrakt, wasserbasiert (1,09 Gew.-% trans-epsilon-Viniferin) | 0,50 | 0,25 | 1,00 |
| Taurin | 1,00 | 0,50 | 2,00 |
| Calmosensine | 1,00 | 2,00 | - |
| Keltrol SF | 0,20 | 0,20 | 0,20 |
| N,N-Bis-(2-hydroxyethyl)harnstoff | 4,30 | 2,50 | 8,60 |
| Perfume | 0,30 | 0,30 | 0,30 |
| Natrium Ascorbylphosphat | 1,00 | - | 1,00 |
| Keratec Pep | - | 2,00 | - |
| Kreatin | - | - | 1,00 |
| Retinol | 0,10 | - | - |
| Deliner | - | 2,00 | - |
| Wasser | ad 100 | ad 100 | ad 100 |

### 3. Tagescremes:

Die Zusammensetzungen 1 - 5 sind bevorzugte Ausführungsformen von zellverjüngenden Tagescremes für besonders anspruchsvolle Haut mit einem Gehalt an Antiageing-Wirkstoffen.

| | 1 | 2 | 3 | 4 | 5 |
|---|---|---|---|---|---|
| Lipoid S 75-3 | 0,50 | 0,50 | 0,50 | 0,50 | 0,50 |
| Isopropylstearate | 4,00 | 4,00 | 4,00 | 4,00 | 4,00 |
| Dibutyl Adipate | 2,00 | 2,00 | 2,00 | 2,00 | 2,00 |
| Tocopheryl Acetate | 0,50 | 0,50 | 0,50 | 0,50 | 0,50 |
| Cutina MD | 1,00 | 1,00 | 1,00 | 1,00 | 1,00 |
| Behenyl Alcohol | 2,00 | 2,00 | 2,00 | 2,00 | 2,00 |
| Baysilone-Öl M 350 | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerol | 3,00 | 4,50 | 3,00 | 3,00 | 3,00 |
| Hexandiol | 6,00 | 3,00 | - | - | 4,00 |
| Methylparaben | 0,20 | 0,20 | 0,20 | 0,20 | 0,20 |
| Tego Carbomer | 0,30 | 0,30 | 0,30 | 0,30 | 0,30 |
| Algae Extract | 1,00 | - | - | - | - |
| Photosomes | 0,20 | 0,20 | 0,20 | 0,20 | 0,20 |
| Dimethylmethoxy Chromanol | 0,01 | 0,01 | 0,01 | 0,01 | 0,01 |
| Propylenglycol | 5,00 | 5,00 | 5,00 | 5,00 | 5,00 |
| Phytokine | 2,00 | 1,50 | 2,00 | 2,00 | 1,50 |
| Ridulisse C | 0,50 | 0,50 | 0,50 | 0,50 | 0,50 |
| Liftiline | 2,00 | 3,00 | 2,00 | 2,00 | 3,00 |
| Weinrebenextrakt, wasserbasiert (1,09 Gew.-% trans-epsilon-Viniferin) | 1,00 | 1,00 | 0,50 | 1,00 | 0,20 |
| Calmosensine | 1,00 | 2,00 | - | 1,00 | 1,00 |
| Kombuchka | 3,00 | 3,00 | - | - | 2,00 |
| Natrium Ascorbylphosphat | - | - | 1,00 | 1,00 | 1,00 |
| Natrulon RC50DG | - | - | - | - | 1,00 |
| Deliner | - | - | 2,00 | 1,00 | - |
| Matrixyl 3000 | 1,00 | 1,00 | - | - | 1,00 |
| Simulgel NS | 1,50 | 1,50 | 1,50 | 1,50 | 1,50 |
| Sodium Benzoate | - | 0,20 | 0,50 | - | - |
| Phenoxyethanol | 0,50 | - | - | - | - |
| Symdiol 68 | 0,30 | 0,50 | 0,30 | - | - |
| TiO₂ | 0,50 | 0,50 | - | - | - |
| Hydrovance | 2,50 | 4,30 | 8,60 | 8,60 | 8,60 |
| Silymarin Phytosome | 0,30 | - | - | - | 0,50 |
| Ectoin | 0,50 | - | 0,50 | 0,50 | 0,50 |
| Vitamin B6 | - | - | 0,01 | 0,01 | 0,01 |
| Perfume | 0,35 | 0,35 | 0,35 | 0,35 | 0,35 |
| Aqua | ad 100 | ad 100 | ad 100 | ad 100 | ad 100 |

Die Zusammensetzungen 1 - 6 sind bevorzugte Ausführungsformen von zellverjüngenden Pflegecremes mit gel-ähnlicher Konsistenz.

| | 1 | 2 | 3 | 4 | 5 | 6 |
|---|---|---|---|---|---|---|
| 2-Ethylhexylpalmitat | 2,00 | 2,00 | 2,00 | 2,00 | 2,00 | 2,00 |
| Dow Corning 1403 Fluid | 5,00 | 5,00 | 5,00 | 5,00 | 5,00 | 5,00 |
| Synperonic PE L 64 | 2,00 | 2,00 | 2,00 | 2,00 | 2,00 | 2,00 |
| Dow Corning 9040 | 5,00 | 5,00 | 5,00 | 5,00 | 5,00 | 5,00 |
| Dipropylenglykol | 5,00 | 5,00 | 5,00 | 5,00 | 5,00 | 5,00 |
| Tego Carbomer 140 | 0,70 | 0,70 | 0,70 | 0,70 | 0,70 | 0,70 |
| Parfum | 0,35 | 0,30 | 0,35 | 0,35 | 0,30 | 0,30 |
| Phenoxyethanol, rein | 0,40 | 0,40 | 0,40 | 0,40 | 0,40 | 0,40 |
| Natrulon RC-50DG | - | 0,75 | 0,75 | 0,75 | 0,75 | 0,75 |
| Menthyl Lactate | 0,50 | 0,50 | 0,50 | 0,50 | 0,50 | 0,50 |
| Ethanol 96 % DEP vergällt | 5,00 | 5,00 | 5,00 | 5,00 | 5,00 | 5,00 |
| Coffein wasserfrei | 0,50 | - | 0,50 | 0,50 | 0,50 | 0,50 |
| Ridulisse C | - | - | - | 0,50 | - | - |
| Weinrebenextrakt, wasserbasiert (1,09 Gew.-% transepsilon-Viniferin) | 0,50 | 0,50 | 0,50 | 1,00 | 1,00 | 1,00 |
| Simulgel NS | 0,75 | 0,75 | 0,75 | 0,75 | 0,75 | 0,75 |
| Matrixyl | - | - | - | - | 2,00 | - |
| Matrixyl 3000 | - | - | - | - | - | 2,00 |
| Natriumhydroxid Perlen | 0,081 | 0,081 | 0,081 | 0,081 | 0,081 | 0,081 |
| Wasser, vollentsalzt | ad 100,00 | ad 100,00 | ad 100,00 | ad 100,00ad | 100,00 | ad 100,00 |

### 4. Die Zusammensetzungen 1 - 5 sind bevorzugte Ausführungsformen von zellverjüngenden Tagescremes mit höherem Lichtschutzfilter (SPF ca. 10 - 15) für besonders anspruchsvolle Haut mit einem Gehalt an Antiageing-Wirkstoffen.

| | 1 | 2 | 3 | 4 | 5 |
|---|---|---|---|---|---|
| Montanov 68 | 5,00 | 5,00 | 5,00 | 5,00 | 5,00 |
| Myritol318 | 5,00 | 5,00 | 5,00 | 5,00 | 5,00 |
| Cetiol SB 45 | 0,50 | 0,50 | 0,50 | 0,50 | 0,50 |
| Novata AB | 2,00 | 2,00 | 2,00 | 2,00 | 2,00 |
| Stenol 1618 | 1,00 | 1,00 | 1,00 | 1,00 | 1,00 |
| Baysilon M 350 | 0,50 | 0,50 | 0,50 | 0,50 | 0,50 |
| Tocopherylacetat | 0,50 | 0,50 | 0,50 | 0,50 | 0,50 |
| Controx KS | 0,25 | 0,25 | 0,25 | 0,25 | 0,25 |
| Parsol SLX | - | 4,00 | 4,00 | - | - |
| Parsol HS | - | 2,00 | 2,00 | 2,00 | - |
| Neo Heliopan AP | - | - | - | 1,00 | - |
| Parsol 340 | - | - | - | 5,00 | - |
| Uvinul MBC 95 | 2,00 | - | - | - | - |
| Parsol1789 | 1,00 | 1,80 | 1,80 | - | - |
| Propylparaben | 0,20 | 0,20 | 0,20 | 0,20 | 0,20 |
| Tego Carbomer 140 | 0,50 | 0,50 | 0,50 | 0,50 | 0,50 |
| 1,6-Hexandiol | 6,00 | 6,00 | 3,00 | 6,00 | - |
| Talkum | 0,50 | 0,50 | 0,50 | 0,50 | 0,50 |
| Methylparaben | 0,20 | 0,20 | 0,20 | 0,20 | 0,20 |
| Glycerin | 4,50 | 4,50 | 3,00 | 4,50 | 3,00 |
| Dry Flo Plus | 1,00 | 1,00 | 1,00 | 1,00 | 1,00 |
| Natipide 2 PG | 1,00 | 1,00 | 1,00 | 1,00 | 1,00 |
| Phenoxyethanol | 0,40 | 0,40 | 0,40 | 0,40 | 0,40 |
| Weinrebenextrakt, wasserbasiert (1,09 Gew.-% trans-epsilon-Viniferin) | 1,00 | 0,50 | 1,00 | 1,00 | 1,00 |
| Carnitin | - | 0,10 | 0,50 | - | - |
| Matrixyl 3000 | 1,00 | - | - | 4,00 | 2,00 |
| Ridulisse C | - | - | 3,00 | - | 0,50 |
| Parfum Aqua | | | | | |
| | 0,40 ad 100 | 0,40 ad 100 | 0,40 ad 100 | 0,40 ad 100 | 0,40 ad 100 |

### 5. Wasser-in-Öl-Emulsion

Die Zusammensetzungen 1 - 3 sind bevorzugte Ausführungsformen von zellverjüngenden Cremes auf der Basis einer reichhaltigen Wasser-in-ÖI-Emulsion, die in die Haut einmassiert werden kann und dadurch einen zusätzlichen Bonuseffekt hervorruft.

| | 1 | 2 | 3 |
|---|---|---|---|
| Lameform TGI | 3,00 | 3,00 | 3,00 |
| Elfacos ST 37 | 0,50 | 0,50 | 0,50 |
| Microcrystalline Wax | 3,00 | 3,00 | 3,00 |
| Softisan 649 | 1,00 | 1,00 | 1,00 |
| Paraffinöl | 8,00 | 8,00 | 8,00 |
| Vaseline | 2,00 | 2,00 | 2,00 |
| Vitamin E Acetat | 2,00 | 2,00 | 2,00 |
| Methylparaben | 0,30 | 0,30 | 0,30 |
| Propylparaben | 0,30 | 0,30 | 0,30 |
| Isopropylisostearat | 8,00 | 8,00 | 8,00 |
| 1,3-Butylenglycol | 5,00 | 6,00 | 7,00 |
| Weinrebenextrakt, wasserbasiert (1,09 Gew.-% trans-epsilon-Viniferin) | 0,50 | 1,00 | 1,00 |
| Carnitin | - | 0,10 | 0,50 |
| Milchsäure 80%ig | 0,60 | 0,60 | 0,60 |
| Panthenol | 0,5 | 1,0 | 0,5 |
| Tephrosia pupurea Seed Extract | 0,1 | - | - |
| Hydrovance | 2,00 | 4,00 | - |
| Parfum | 0,2 | 0,2 | 0,2 |
| Water | ad 100 | ad 100 | ad 100 |

### 6. Die Zusammensetzungen 1 - 3 sind bevorzugte Ausführungsformen von zellverjüngenden Gesichtswässern, die bevorzugt mit einem Pad, Bausch oder Tuch auf die Haut appliziert werden.

| | 1 | 2 | 3 |
|---|---|---|---|
| Dipropylenglycol | 10,00 | 10,00 | 10,00 |
| Chlorhexidindigluconat | 1,00 | 1,00 | 1,00 |
| Synperonic PE7 L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil / Trideceth 9 / Propylene Glycol | 0,50 | 0,50 | 0,50 |
| Caomint | 0,50 | 0,50 | 0,50 |
| Weinrebenextrakt, wasserbasiert (1,0 Gew.-% trans-epsilon-Viniferin) | 1,00 | 0,25 | 0,50 |
| Ridulisse C | 1,00 | - | - |
| Fucogel 1000 | - | - | 1,00 |
| Betain | - | 1,00 | - |
| Parfum | 0,20 | 0,20 | 0,20 |
| Aqua | ad 100 | ad 100 | ad 100 |

### 7. Die Zusammensetzungen 1 - 3 sind bevorzugte Ausführungsformen von zellverjüngenden Cremes auf der Basis einer reichhaltigen und dennoch nicht-fettenden Wasser-in-Silicon-Emulsion, die in die Haut einmassiert werden kann.

| | 1 | 2 | 3 |
|---|---|---|---|
| Belsil DM 100 | 2,50 | 2,50 | 2,50 |
| Dow Corning 245 Fluid | 25,00 | 25,00 | 25,00 |
| Abil EM 90 | 2,00 | 2,00 | 2,00 |
| Natriumchlorid | 2,00 | 2,00 | 2,00 |
| Symdiol 68 | 0,30 | 0,30 | 0,30 |
| Phenoxyethanol | 0,40 | 0,40 | 0,40 |
| Weinrebenextrakt, wasserbasiert (1,09 Gew.-% trans-epsilon-Viniferin) | 1,00 | 1,00 | 1,00 |
| Ridulisse C | - | 2,00 | 1,00 |
| Rhamnosoft | 2,00 | - | - |
| Hydrovance | - | 3,00 | - |
| Folsäure | - | - | 0,10 |
| Parfum | 0,30 | 0,30 | 0,30 |
| Aqua | ad 100 | ad 100 | ad 100 |

### 8. Beispielserie:

Die Zusammensetzungen 1 - 4 sind bevorzugte Ausführungsformen von zellverjüngenden Tagescremes für besonders anspruchsvolle Haut mit einem erhöhten Gehalt an diversen Antiageing-Wirkstoffen.

| | 1 | 2 | 3 | 4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Baysilone-ÖI M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Algenextrakt | 1,00 | - | - | - |
| Symdiol 68 | 0,30 | 0,50 | - | - |
| DSH-CN | 5,00 | 5,00 | 5,00 | 5,00 |
| Hydrovance | 4,30 | 2,50 | 8,60 | 10,00 |
| Weinrebenextrakt, wasserbasiert (1,09 Gew.-% trans-epsilon-Viniferin) | 1,00 | 2,00 | 1,50 | 2,00 |
| Natrulon RC 50 DG | - | - | 1,00 | - |
| Phytokine | 2,00 | | - | - |
| Matrixyl 3000 | - | 2,00 | - | - |
| Ridulisse C | - | - | 2,00 | 2,50 |
| Argireline | 1,00 | - | - | - |
| Sepilift DPHP | - | 0,10 | - | - |
| Ederline H | 2,00 | - | - | - |
| Perfume | 0,10 | 0,10 | 0,10 | 0,10 |
| Aqua | ad 100 | ad 100 | ad 100 | ad 100 |

### 9. Tagescreme:

Die folgende Zusammensetzung ist eine weitere bevorzugte Ausführungsform einer zellverjüngenden erfindungsgemäßen Zusammensetzung.

| | |
|---|---|
| Lipoid S 75-3 | 1,000000 |
| Isopropylstearat | 2,000000 |
| Cetiol B | 4,000000 |
| Vitamin E Acetat | 0,500000 |
| Cutina MD | 1,000000 |
| Cetearyl Alcohol | 2,500000 |
| NOVATA AB PH | 0,500000 |
| Behenylalkohol | 3,500000 |
| Silikonöl 350 cs | 1,500000 |
| Propylparaben | 0,200000 |
| Dow Corning 9040 | 2,000000 |
| Glycerin 86% | 8,000000 |
| Hexandiol-1,6 | 6,000000 |
| Methylparaben | 0,200000 |
| Talkum | 3,000000 |
| Tego Carbomer 140 | 0,400000 |
| Hydroglycolic Extract of Caviar | 1,000000 |
| Weinrebenextrakt, wasserbasiert (1,09 Gew.-% trans-epsilon-Viniferin) | 0,500000 |
| Ederline L | 1,000000 |
| DSH-C N | 3,000000 |
| Photosomes | 0,200000 |
| Lipochroman-6 | 0,010000 |
| Propandiol-1,2 | 4,000000 |
| Matrixyl 3000 | 3,000000 |
| Phenoxyethanol, rein | 0,400000 |
| Parfum | 0,200000 |
| RONASHERE LDP | 1,000000 |
| FD&C Yellow No. 6 | 0,000700 |
| Simulgel NS | 1,500000 |
| Natriumhydroxid | 0,040000 |
| Wasser, vollentsalzt | ad 100,00000 |

### 10. Nachtcreme:

Die folgende Zusammensetzung ist eine weitere bevorzugte Ausführungsform einer zellverjüngenden erfindungsgemäßen Zusammensetzung.

| | |
|---|---|
| Montanov 68 | 3,000000 |
| Capryl-/Caprinsäure-Triglycerid | 2,000000 |
| Cetiol SN | 8,000000 |
| Safloröl raffiniert | 3,500000 |
| Cetearyl Alcohol | 2,500000 |
| Cutina MD | 1,700000 |
| Behenylalkohol | 3,000000 |
| Cetiol SB 45 | 4,000000 |
| NOVATA AB PH | 0,500000 |
| Silikonöl 350 cs | 2,500000 |
| Controx KS | 0,050000 |
| Propylparaben | 0,200000 |
| Glycerin 86% | 9,000000 |
| Hexandiol-1,6 | 6,000000 |
| Methylparaben | 0,200000 |
| Tego Carbomer 140 | 0,300000 |
| Weinrebenextrakt, wasserbasiert (1,09 Gew.-% transepsilon-Viniferin) | 2,000000 |
| Phenoxyethanol, rein | 0,400000 |
| Ultrasomes | 0,100000 |
| Matrixyl 3000 | 3,000000 |
| Parfum | 0,100000 |
| FD&C Yellow No. 6 | 0,000700 |
| Aluminiumstärkeoctenylsuccinat | 1,800000 |
| Simulgel EPG | 0,400000 |
| Natriumhydroxid | 0,031000 |
| Wasser, vollentsalzt | ad 100,000000 |

### 11. Hautcremes auf Basis einer Lipoprotein-Creme

Die Zusammensetzungen 1 - 6 sind bevorzugte Ausführungsformen von zellverjüngenden Cremes mit besonderer Hautverträglichkeit und einem Gehalt an diversen Antiageing-Wirkstoffen, auf Basis einer besonders milden Emulsionsbasis mit einem natürlichen Protein-Emulgator (Hibiscin^{®} HP LS 9198).

| | 1 | 2 | 3 | 4 | 5 | 6 |
|---|---|---|---|---|---|---|
| Myritol^{®} PC | 3,5 | 3,5 | 3,5 | 3,5 | 3,5 | 3,5 |
| Lanette^{®} 22 | 3,0 | 3,0 | 3,0 | 3,0 | 3,0 | 3,0 |
| Cutina^{®} GMS-V | 3,0 | 3,0 | 3,0 | 3,0 | 3,0 | 3,0 |
| Stenol^{®} 16/18 | 2,0 | 2,0 | 2,0 | 2,0 | 2,0 | 2,0 |
| Distelöl | 3,0 | 3,0 | 3,0 | 3,0 | 3,0 | 3,0 |
| Controx^{®} KS | 0,05 | 0,05 | 0,05 | 0,05 | 0,05 | 0,05 |
| Baysilon^{®} M350 | 1,0 | 1,0 | 1,0 | 1,0 | 1,0 | 1,0 |
| Isopropylstearat | 6,0 | 6,0 | 6,0 | 6,0 | 6,0 | 6,0 |
| Propylparaben | 0,2 | 0,2 | 0,2 | 0,2 | 0,2 | 0,2 |
| 1,3-Butylenglycol | 5,0 | 5,0 | 5,0 | 5,0 | 5,0 | 5,0 |
| 1,6-Hexandiol | 5,0 | 5,0 | 5,0 | 5,0 | 5,0 | 5,0 |
| Methylparaben | 0,2 | 0,2 | 0,2 | 0,2 | 0,2 | 0,2 |
| Hibiscin^{®} HP LS 9198 | 3,0 | 3,0 | 3,0 | 3,0 | 3,0 | 3,0 |
| Citronensäure | 0,1 | 0,1 | 0,1 | 0,1 | 0,1 | 0,1 |
| Simulgel^{®} NS | 2,0 | 2,0 | 2,0 | 2,0 | 2,0 | 2,0 |
| Pearl Protein Extract | - | - | 2,00 | - | - | 1,00 |
| Weinrebenextrakt, wasserbasiert (1,09 Gew.-% trans-epsilon-Viniferin) | 2,0 | 1,0 | 1,0 | 1,0 | 1,0 | 1,0 |
| Ridulisse C | 1,00 | 0,50 | 1,00 | - | - | - |
| Carnitin | 0,20 | - | - | 0,50 | 0,30 | 0,30 |
| Matrixyl 3000 | 3,00 | - | - | - | 2,00 | |
| Aqua | ad 100 | ad 100 | ad 100 | ad 100 | ad 100 | ad 100 |

### 12. Reinigungsgele mit zellverjüngendem Effekt:

Die Zusammensetzungen 1 - 3 sind bevorzugte Ausführungsformen von Reinigungsgelen mit einem zellverjüngenden Effekt zur Komplettierung der Hautpflege.

| | 1 | 2 | 3 |
|---|---|---|---|
| Carbomer | 1,40 | 1,40 | 1,40 |
| 1,3-Butylenglycol | 4,00 | 4,00 | 4,00 |
| Sodium Benzoate | 0,40 | 0,40 | 0,40 |
| Plantaren^{®} 1200 | 7,50 | 7,50 | 7,50 |
| Dehyton^{®} K | 3,40 | 3,40 | 3,40 |
| Texapon^{®} SB 3 | 5,00 | 5,00 | 5,00 |
| Cetiol^{®} HE | 0,50 | 0,50 | 0,50 |
| Lamesoft^{®} PO 65 | 5,00 | 5,00 | 5,00 |
| Controx KS | 0,05 | 0,05 | 0,05 |
| Sodium pyrrolidone carboxylic acid | 1,60 | 1,60 | - |
| Pantolactone | 1,00 | 1,00 | - |
| Tetrasodium EDTA | 0,25 | 0,25 | 0,25 |
| Sodium Lactate | 1,80 | | |
| Panthenol | 0,50 | 0,50 | 0,50 |
| Weinrebenextrakt, wasserbasiert (1,09 Gew.-% trans-epsilon-Viniferin) | 1,0 | 1,0 | 1,0 |
| Ridulisse C | 1,00 | - | - |
| Carnitin | 0,20 | - | 0,20 |
| Perfume | 0,40 | 0,40 | 0,40 |
| Aqua | ad 100 | ad 100 | ad 100 |

### 13. Beispiele für Zusammensetzungen zum Tränken von Tüchern

| | Bestandteile | 1 | 2 | 3 | 4 |
|---|---|---|---|---|---|
| Phase 1 | PEG-40 Hydrogenated Castor Oil | 0,1 | 0,1 | 0,1 | - |
| | Trideceth-9 | 0,1 | 0,1 | 0,1 | - |
| | Parfum | 0,3 | 0,1 | 0,1 | 0,1 |
| Phase 2 | Hexyllaurat | 2,0 | - | - | - |
| | Dicaprylylcarbonat | - | - | 2,0 | - |
| | Dipropylenglycol | - | 2,0 | - | - |
| Phase 3 | Allantoin | 0,5 | - | - | - |
| | Citronensäure (Monohydrat) | 1,5 | 1,5 | 1,5 | 1,5 |
| | Trinatriumcitrat (Dihydrat) | 2,0 | 2,0 | 2,0 | 2,0 |
| | Decylglucosid | - | - | - | 2,0 |
| | Pemulen TR 1 | - | - | - | 0,2 |
| | Weinrebenextrakt, wasserbasiert (1,09 Gew.-% trans-epsilon-Viniferin) | 3,0 | 3,0 | 3,0 | 1,0 |
| | Ridulisse C | 1,00 | 0,50 | 1,00 | - |
| | Carnitin | 0,20 | - | - | 0,50 |
| | Wasser | ad 100 | ad 100 | ad 100 | ad 100 |

Mit diesen Tränkzusammensetzungen wurden verschiedene Viskose-Vliesstoffe ausgerüstet. Es wurden gelochte, ungelochte, genoppte, einlagige, zweilagige und dreilagige Vliese ausgerüstet. Die Tücher wurden sowohl als feuchte Tücher als auch als trockene Tücher (das heißt, nach dem Ausrüsten wurden die Tücher bis auf einen Restwassergehalt kleiner 10 Gew.-%, bevorzugt kleiner 5 Gew.-%, getrocknet, um direkt vor Gebrauch mit einer kleinen Menge an Wasser wieder befeuchtet zu werden oder auf feuchter Haut angewendet zu werden) verwendet.

### Matrixpflaster mit zellverjüngendem Effekt

| | 1 | 2 | 3 | 4 | 5 |
|---|---|---|---|---|---|
| DURO-TAK^{®} 387-2516 | 76 | 76 | 76 | 76 | 76 |
| Aloe Vera Gel | 1 | - | 1 | - | - |
| Propylenglycolmonooleat | 5 | 5 | - | - | - |
| Tween^{®}-80 | - | - | 5 | 3 | 5 |
| Natriumcitrat | 0,1 | 0,1 | 0,1 | 0,1 | 0,1 |
| Controx^{®} KS | 0,05 | 0,05 | 0,05 | 0,05 | 0,05 |
| Weinrebenextrakt, wasserbasiert (1,09 Gew.-% trans-epsilon-Viniferin) | 3,0 | 3,0 | 3,0 | 1,0 | 3,0 |
| Ridulisse C | 1,00 | 0,50 | 1,00 | - | - |
| Carnitin | 0,20 | - | - | 0,50 | 0,30 |
| Matrixyl 3000 | 3,00 | - | - | - | 2,00 |
| Wasser | ad 100 | ad 100 | ad 100 | ad 100 | ad 100 |

### Reservoirpflaster mit zellverjüngendem Effekt

| Bestandteile des Wirkstoffreservoirs | 1 | 2 |
|---|---|---|
| Weinrebenextrakt, wasserbasiert (1,09 Gew.-% trans-epsilon-Viniferin) | 2,0 | 3,0 |
| Ultrasomes | 1,0 | 1,0 |
| Ridulisse C | - | 0,50 |
| Carnitin | 0,50 | - |
| Ederline H | 1,0 | 1,0 |
| Carbopol^{®} 980 | 0,5 | - |
| Pemulen^{®}TR 1 | - | 0,5 |
| NaOH | 0,1 | 0,1 |
| Titandioxid | 0,2 | 0,2 |
| Ethanol | 1,0 | 1,0 |
| Polyvinylalkohol | 2,0 | 1,0 |
| Carboxymethylcellulose | 1,0 | 0,5 |
| Glycerin | 10 | 20 |
| Sorbitol | 7,0 | 5,0 |
| 1,3-Butandiol | 3,0 | 3,0 |
| Tween^{®}-80 | 0,05 | 0,05 |
| Paraffinöl | 5,0 | 2,0 |
| Natriumcitrat | 0,1 | 0,1 |
| Controx KS | 0,05 | 0,05 |
| Wasser | ad 100 | ad 100 |

Die Bestandteile der Wirkstoffreservoir-Rezepturen Nr. 1 bzw. 2 wurden miteinander vermischt, so dass eine gelartige Masse entstand. Mit dieser Masse wurden Pflasterträger beschichtet, so dass sogenannte Reservoirpflaster erhalten wurden. Diese Pflaster werden sowohl auf die trockene Haut als auch auf die angefeuchtete Haut gelegt und verbleiben dort für eine Zeit von wenigen Sekunden bis einigen Stunden.

Die nachfolgendenRezepturen sind bevorzugte Beispiele für erfindungsgemäße zellverjüngende Körpercremes:

| | | |
|---|---|---|
| Paraffinum Liquidum | 20,00 | 20,00 |
| Arlacel 165 | 4,00 | 4,00 |
| Eutanol G | 1,00 | 1,00 |
| Cetiol SN | 1,50 | 1,50 |
| Cremophor A 6 | 2,00 | 2,00 |
| Behenylalkohol | 1,60 | 1,60 |
| Silikonöl 350 cs | 3,00 | 3,00 |
| Brij 721 VP | 0,40 | 0,40 |
| Propylparaben | 0,20 | 0,20 |
| Controx KS | 0,25 | 0,25 |
| Propandiol-1,2 | 3,00 | 3,00 |
| Glycerin 86% pflanzlich | 5,00 | 5,00 |
| Polyethylenglykol MG 400 | 1,70 | 1,70 |
| Methylparaben | 0,20 | 0,20 |
| Phenoxyethanol, rein | 0,50 | 0,50 |
| Carbopol ETD 2020 | 0,50 | 0,50 |
| Natriumhydroxid | 0,03 | 0,03 |
| Citronensäure Monohydrat | 0,10 | 0,10 |
| Trinatriumcitrat Dihydrat | 0,10 | 0,10 |
| Weinrebenextrakt, wasserbasiert (1,09 Gew.-% trans-epsilon-Viniferin) | 3,00 | 3,00 |
| Sensiva SC 50 (Octoxyglycerin) | 0,50 | 0,50 |
| Carnitin | 0,20 | 0,20 |
| Ergothionein | - | 0,10 |
| Parfum | 0,30 | 0,30 |
| Simulgel NS | 1,00 | 1,00 |
| Wasser | ad 100,00 | ad 100,00 |

### Liste der verwendeten Rohstoffe

| Handelsname | INCl-Bezeichnung | Lieferant/Hersteller |
|---|---|---|
| | | |
| Abil EM 90 | CETYL PEG/PPG-10/1 DIMETHICONE | Degussa |
| Argireline | Acetyl-Hexapeptide-3 | Lipotec |
| Arlacel 165 | Glyceryl Stearate, PEG-100 Stearate | Uniqema |
| Baysilone-ÖI M 350 | Dimethicone | GE Bayer Silicones |
| Belsil DM 100 | Dimethicone | Wacker |
| Brij 721 | STEARETH-21 | Uniqema |
| Calmosensine | BUTYLENE GLYCOL, WATER, LAURETH-3, HYDROXYETHYLCELLULOSE, Acetyl Dipeptide-1 Cetylester | Sederma |
| Caomint | PROPYLENE GLYCOL, AQUA (WATER), MENTHA PIPERITA (PEPPERMINT) LEAF EXTRACT, THEOBROMA CACAO (COCOA) EXTRACT | Solabia |
| Carbopol 980 | Carbomer | Noveon |
| Carbopol ETD 2020 | Acrylates/C10-30 Alkyl Acrylate Crosspolymer | Noveon |
| Cetiol B | Dibutyl Adipate | Cognis |
| Cetiol^{®} HE | PEG-7 Glyceryl Cocoate | Cognis |
| Cetiol^{®} SB 45 | Butyrospermum parkii (Shea butter) | Cognis |
| Cetiol^{®} SN | Cetearyl Isononanoate | Cognis |
| Controx KS | Tocopherol, Hydrogenated Palm Glycerides Citrate | Cognis |
| Cremophor A 6 | CETEARETH-6, STEARYL ALCOHOL | BASF |
| Cutina CBS | Glyceryl Stearate / Cetearyl Alcohol / Cetyl □alpitate / Cocoglycerides im Gewichtsverhältnis 7:1:1:1 | Cognis |
| Cutina GMS-V | Glyceryl Stearate (Mischung aus Glycerylmono- und distearat) | Cognis |
| Cutina MD | Glyceryl Stearate (Mischung aus Glycerylmono- und distearat) | Cognis |
| Dehyton K | AQUA (WATER), COCAMIDOPROPYL BETAINE (29-32 Gew.-%) | Cognis |
| Deliner | Zea Mays (Corn) Kernel Extract, Butylene Glycol, Xanthan Gum | Coletica |
| Dow Corning 1403 Fluid | Cyclomethicone (86 - 88 Gew.-%), Dimethiconol (12 - 14 Gew.-%) | Dow Corning |
| Dow Corning 1501 Fluid | Cyclomethicone (85 - 86 Gew.-%), Dimethiconol (14-15 Gew.-%) | Dow Corning |
| Dow Corning 9040 | Cyclomethicone/ Dimethicone Crosspolymer (87-88%/12-13 %) | Dow Corning |
| Dow Corning^{®}245 Fluid | Cyclomethicone | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinylpyrrolidon | National Starch and Chemical |
| Ederline H | PEG-40 Hydrogenated Castor Oil, PPG-2-Ceteareth-9, Pyrus Malus (Apple) Fruit Extract | Seporga |
| Ederline L | Hexyldecanol, Pyrus Malus (Apple) Fruit Extract | Seporga |
| Elfacos ST 37 | PEG-45/Dodecyl Glycol Copolymer | Akzo Nobel |
| Eumulgin B 3 | Ceteareth-30 | Cognis |
| Eutanol G | OCTYLDODECANOL | Cognis |
| Fucogel 1000 | Biosaccharide Gum-1 | Solabia |
| Glistin | Water, L-Glutamylaminoethyl indole | Exsymol |
| Hibiscin^{®} HP-LS-9198 | Water, Hibiscus esculentus Seed Extract, Phenoxyethanol | Laboratoires Sérobiologiques |
| Hydagen CMF | Chitosan Glycolate | Cognis |
| Hydrovance | Wasser, Bis-N,N'-(2-Hydroxyethyl)harnstoff, Harnstoff, Ammoniumlactat | National Starch |
| Keltrol SF | Xanthan Gum | Kelco |
| Keratec Pep | Water, Hydrolyzed Keratin | Croda |
| Kombuchka | Saccharomyces/Xylinum/Black Tea Ferment, Glycerin, Hydroxyethylcellulose | Sederma |
| Lameform TGI | Polyglyceryl-3 Diisostearate | Cognis |
| Lamesoft^{®} PO 65 | Coco-Glucoside, Glyceryl Oleate, Water | Cognis |
| LANETTE^{®} 22 | Behenyl Alcohol | Cognis |
| Liftiline | Aqua, Hydrolyzed Wheat Protein (7 - 10 Gew.-% Aktivsubstanz) | Silab |
| Lipochroman 6 | DIMETHYLMETHOXY CHROMANOL | Lipotec |
| Lipoid S 75-3 | Aqua, Lecithine | Lipoid GmbH |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Myritol 318 | Caprylic/Capric Triglyceride | Cognis |
| MYRITOL^{®} PC | PROPYLENE GLYCOL DICAPRYLATE/DICAPRATE | Cognis |
| Natipide 2 PG | Aqua, Propylene Glycol, Lecithin | Phospholipid GmbH |
| Natrulon RC 50 DG | WATER, CARNITINE, POLYGLYCERIN-10 | Lonza |
| Neo Heliopan AP | Disodium Phenyl Dibenzimidazole Tetrasulfonate | Symrise |
| Novata AB | Cocoglycerides | Cognis |
| Parsol 1789 | BUTYL METHOXYDIBENZOYLMETHANE | DSM |
| Parsol 340 | Octocrylene | DSM |
| Parsol HS | PHENYLBENZIMIDAZOLE SULFONIC ACID | DSM |
| Parsol SLX | Polysilicone-15 | DSM |
| Pearl Protein Extract | Aqua, Hydrolyzed Conchiolin Protein | Maruzen |
| Pemulen TR 1 | Acrylates/C10-30 Alkyl Acrylate Crosspolymer | Noveon |
| Photosomes | Aqua, Lecithine, Plancton Extract | AGI Dermatics |
| Phytokine | Aqua, Butylene Glycol, HYDROLYZED SOY PROTEIN, Methylparaben, Ethylparaben, Propylparaben (0,4 - 0,8 Gew.-% Aktivsubstanz) | Coletica |
| Plantaren^{®} 1200 | Lauryl Glucoside, ca. 50 % Aktivsubstanz | Cognis |
| Rhamnosoft | Biosaccharide Gum-2 | Solabia |
| Ridulisse C | Water, HYDROLYZED SOY PROTEIN (3 - 5 Gew.-% Aktivsubstanz) | Silab |
| Ronasphere^{®} LDP | SILICA, Cl 77891 (TITANIUM DIOXIDE), Cl 77491 (IRON OXIDES) | Merck KGaA |
| Sensiva^{®} SC 50 | 2-Ethylhexylglycerinether | Schülke & Mayr |
| Sepigel^{®}305 | Aqua (Water) Polyacrylamide, C₁₃-C₁₄ Isoparaffin, Laureth-7 (Aktivsubstanz Verdickerpolymer ca. 45 - 49 Gew.-%) | SEPPIC |
| Sepilift DPHP | Dipalmitoyl Hydroxyproline | Seppic |
| Sepivinol R | Wine Extract (Polyphenolreicher Extrakt aus Rotwein) | Seppic |
| Silymarin Phytosome | Silybum Marianum Extract and Phospholipids | Indena SpA |
| Simulgel EPG | Aqua (Water)/Sodium Acrylate / Sodium Acryloyldimethyl Taurate Copolymer / Polyisobutene / Caprylyl/Capryl Glucoside | Seppic |
| Simulgel NS | Aqua (Water)/Hydroxyethyl Acrylate / Sodium Acryloyldimethyl Taurate Copolymer / Squalane / Polysorbate 60 | Seppic |
| Softisan 649 | Bis-Diglyceryl Polyacyladipate-2 | Sasol |
| Stenol 16/18 | Cetearylalkohol | Cognis |
| Symdiol 68 | 1,2-Octandiol, 1,2-Hexandiol | Symrise |
| Synperonic PE L 64 | Poloxamer-184 | Uniqema |
| Tego Carbomer 140 | Carbomer | Degussa |
| Texapon^{®} SB 3 | Aqua, Disodium Laureth Sulfosuccinate, (ca. 40 % Aktivsubstanz), Citric Acid | Cognis |
| Tween^{®}-80 | Polysorbate-80 | |
| Ultrasomes | Aqua, Lecithine, Micrococcus Luteus Extract | AGI Dermatics |
| Uvinul MBC 95 | 4-METHYLBENZYLIDENE CAMPHOR | BASF |

## Patentansprüche

1. Nicht-therapeutische, kosmetische Verwendung von mindestens einer Verbindung, ausgewählt aus den Hydroxystilben-Oligomeren, deren Alkylethern und/oder deren Estern,
- zur Verjüngung und/oder Revitalisierung der Zellen, insbesondere der Zellen der Epidermis und/oder der Dermis, oder
- zur Verzögerung des Übergangs der Zellen, insbesondere der Zellen der Epidermis und/oder der Dermis, aus dem proliferativen Status in den ruhenden, seneszenten Status, oder
- zur Verringerung des Anteils der Zellen, insbesondere der Zellen in der Epidermis und/oder der Dermis, die sich im ruhenden, seneszenten Status befinden, oder
- um der Haut einen jüngeren Phänotyp zu verleihen.

2. Verwendung von mindestens einer Verbindung, ausgewählt aus den Hydroxystilben-Oligomeren, deren Alkylethern und/oder deren Estern, zur Herstellung einer Zusammensetzung
- zur Verjüngung von Zellen, insbesondere der Zellen der Epidermis und/oder der Dermis, oder
- zur Verzögerung des Übergangs von Zellen, insbesondere der Zellen der Epidermis und/oder der Dermis, aus dem proliferativen Status in den ruhenden, seneszenten Status, oder
- zur Verringerung des Anteils von Zellen, insbesondere der Zellen in der Epidermis und/oder der Dermis, die sich im ruhenden, seneszenten Status befinden, oder
- um der Haut einen jüngeren Phänotyp zu verleihen.

3. Verwendung gemäß Anspruch 1 oder 2, **dadurch gekennzeichnet, dass** die mindestens eine Verbindung, ausgewählt aus den Hydroxystilben-Oligomeren, deren Alkylethern und/oder deren Estern, in einer zur Verabreichung auf topischem oder oralem Wege und/oder zur Injektion geeigneten Zusammensetzung konfektioniert ist.

4. Verwendung gemäß einem der vorstehenden Ansprüche, **dadurch gekennzeichnet, dass** das mindestens eine Hydroxystilben-Oligomer gebildet ist aus Hydroxystilben-Monomeren der allgemeinen Formel (RESV-Ia) oder (RESV-Ib) : 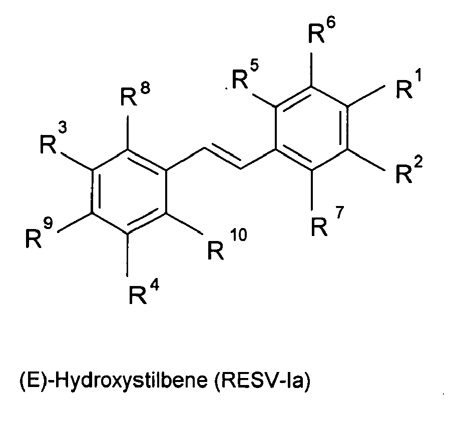 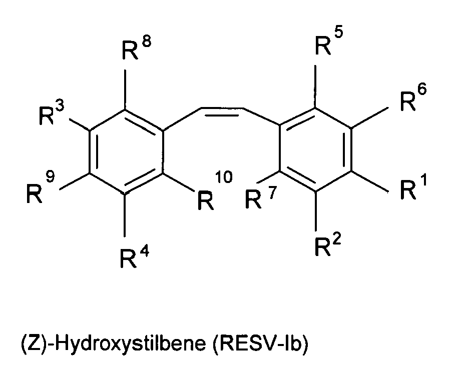 wobei die Reste R¹ bis R¹⁰ unabhängig voneinander ein Wasserstoffatom, eine OH-Gruppe, eine Alkylgruppe, die bevorzugt eine C₁-C₄-Alkylgruppe darstellt, eine Alkoxygruppe, die bevorzugt einen C₁-C₄-Alkylrest trägt, ein Glycosid-Rest, eine ganz oder teilweise neutralisierte Phosphatgruppe -OPO₃, eine ganz oder teilweise neutralisierte Sulfatgruppe -OSO₃ oder eine Estergruppe -OCOR' mit R'COOH = organische Säure sein können, wobei mindestens einer der Reste R¹ bis R¹⁰ ausgewählt ist aus einer OH-Gruppe, einer Alkoxygruppe, die bevorzugt einen C₁-C₄-Alkylrest trägt, einem Glycosid-Rest, einer ganz oder teilweise neutralisierten Phosphatgruppe -OPO₃, einer ganz oder teilweise neutralisierten Sulfatgruppe -OSO₃ und einer Estergruppe -OCOR' mit R'COOH = organische Säure.

5. Verwendung gemäß einem der vorstehenden Ansprüche, **dadurch gekennzeichnet, dass** das mindestens eine Hydroxystilben-Oligomer ausgewählt ist aus Dimeren, Trimeren, Tetrameren, Pentameren und Hexameren von Hydroxystilbenen.

6. Verwendung gemäß einem der vorstehenden Ansprüche, **dadurch gekennzeichnet, dass** das mindestens eine Hydroxystilben-Oligomer aus folgenden Hydroxystilben-Monomeren gebildet ist:
4'-Hydroxystilben (R¹ = OH),
2',4'-Dihydroxystilben (R¹ = R⁵ = OH),
3',4'-Dihydroxystilben (R² = R⁵ = OH),
4,4'-Dihydroxystilben (R¹ = R⁹ = OH),
3,5- Dihydroxystilben (Pinosylvin) (R³ = R⁴ = OH),
3-Hydroxy-5-methoxystilben (Pinosylvinmonomethylether, R³ = OH, R⁴ = -OCH₃)
2',4',4-Trihydroxystilben (R¹ = R⁵ = R⁹ = OH),
3',4',4-Trihydroxystilben (R² = R⁵ = R⁹ = OH),
2,4,4'-Trihydroxystilben (R¹ = R⁸ = R⁹ = OH),
3,4,4'-Trihydroxystilben (R¹ = R³ = R⁹ = OH),
3,4',5-Trihydroxystilben (Resveratrol) (R¹ = R³ = R⁴ = OH),
2',3,4-Trihydroxystilben (R¹ = R⁵ = R⁶ = OH),
2,3',4-Trihydroxystilben (R⁵ = R⁶ = R⁸ = OH),
2',2,4'-Trihydroxystilben (R¹ = R⁵ = R⁸ = OH),
2,4,4',5-Tetrahydroxystilben (R¹ = R⁴ = R⁸ = R⁹ = OH),
2',3,4',5-Tetrahydroxystilben (R¹ = R³ = R⁴ = R⁵ = OH),
2,2',4,4'-Tetrahydroxystilben (R¹ = R⁵ = R⁸ = R⁹ = OH),
3,3',4',5-Tetrahydroxystilben (Piceatannol) (R¹ = R³ = R⁴ = R⁶ = OH),
2,3',4,4'-Tetrahydroxystilben (R¹ = R⁶ = R⁸ = R⁹ = OH),
3,3',4,4'-Tetrahydroxystilben (R¹ = R³ = R⁶ = R⁹ = OH),
3,3',4',5,5'-Pentahydroxystilben (R¹ = R² = R³ = R⁴ = R⁶ = OH),
2,2',4,4',6-Pentahydroxystilben (R¹ R⁵ = R⁸ = R⁹ = R¹⁰ = OH),
2,3',4,4',6-Pentahydroxystilben (R¹ = R⁶ = R⁸ = R⁹ = R¹⁰ = OH),
2,2',4,4',6,6'-Hexahydroxystilben (R¹ = R⁵ = R⁷ = R⁸ = R⁹ = R¹⁰ = OH),
wobei für die nicht genannten R^{x} = H gilt, das heißt, alle übrigen Substituenten R stellen ein Wasserstoffatom dar, und wobei alle Verbindungen sowohl in der (E)-Form als auch in der (Z)-Form vorliegen.

7. Verwendung gemäß einem der vorstehenden Ansprüche, **dadurch gekennzeichnet, dass** das mindestens eine Hydroxystilben-Oligomer ausgewählt ist aus Oligomeren der (E)-Hydroxystilbene.

8. Verwendung gemäß einem der vorstehenden Ansprüche, **dadurch gekennzeichnet, dass** das mindestens eine Hydroxystilben-Oligomer ausgewählt ist aus Dimeren der (E)-Hydroxystilbene.

9. Verwendung gemäß einem der vorstehenden Ansprüche, **dadurch gekennzeichnet, dass** das mindestens eine Hydroxystilben-Oligomer ausgewählt ist aus trans-epsilon-Viniferin, Vitisin E und Vitisin D sowie Mischungen hiervon.

10. Verwendung gemäß einem der vorstehenden Ansprüche, **dadurch gekennzeichnet, dass** die mindestens eine Verbindung, ausgewählt aus den Hydroxystilben-Oligomeren, deren Alkylethern und/oder deren Estern, in einer Gesamtmenge von 0,00001 - 10 Gew.-%, bevorzugt 0,0001 - 5 Gew.-%, besonders bevorzugt 0,001 - 4 Gew.-%, außerordentlich bevorzugt 0,005 - 1 Gew.-%, jeweils bezogen auf das Gesamtgewicht der Zusammensetzung, verwendet wird.

11. Zusammensetzung, enthaltend in einem geeigneten Träger mindestens ein Hydroxystilben-Oligomer oder einen Alkylether hiervon oder einen Ester hiervon in Kombination mit mindestens einem Wirkstoff, ausgewählt aus
- Aporphin-Alkaloiden,
- Purin und/oder Purinderivaten,
- natürlichen Betainverbindungen,
- alkyl- oder hydroxyalkylsubstituierten Harnstoffverbindungen,
- Monomeren, Oligomeren und Polymeren von Aminosäuren, N-C₂-C₂₄-Acylamino-säuren und/oder den Estern und/oder den physiologisch verträglichen Salzen dieser Substanzen,
- Polysacchariden,
- sowie Mischungen dieser Wirkstoffe.

12. Zusammensetzung gemäß Anspruch 11, **dadurch gekennzeichnet, dass** mindestens ein die Kollagensynthese stimulierender Wirkstoff enthalten ist, ausgewählt aus
- Retinoiden, insbesondere Retinol und C₂-C₂₂-Fettsäureestern des Retinols,
- Kombucha,
- Olivenblattextrakten (Olea Europaea (Olive) Leaf Extract),
- Oleanolsäure,
- Oleanol,
- Grüntee-Extrakten (Camellia Sinensis),
- Kreatin,
- sowie Mischungen der vorgenannten Substanzen.

13. Zusammensetzung gemäß einem der Ansprüche 11 oder 12, **dadurch gekennzeichnet, dass** mindestens ein die Interaktion zwischen der extrazellulären Matrix und den Fibroblasten erhöhender und/oder verbessernder Wirkstoff enthalten ist, ausgewählt aus
- Apfelkernextrakten (Pyrus Malus (Apple) Fruit Extract),
- Lotuskeim-Extrakten (Nelumbo Nucifera Germ Extract),
- Extrakten aus Maiskörnern (Zea Mays (Corn) Kernel Extract),
- Extrakten aus Schwarzen Holunderblüten (Sambucus Nigra Flower Extract),
- Mischungen aus mindestens einem Extrakt aus Kakaobohnen (Theobroma cacao) und mindestens einem Extrakt aus den Blättern der Pfefferminze (Mentha piperita),
- Resveratrol, Resveratrolethern und Resveratrolestern,
- Isoflavonoiden und Isoflavonoid-reichen Pflanzenextrakten,
- Dihydroquercetin (= Taxifolin),
- sowie Mischungen hiervon.

14. Zusammensetzung gemäß einem der Ansprüche 11 - 13, **dadurch gekennzeichnet, dass** mindestens ein weiterer Wirkstoff enthalten ist, ausgewählt aus
- DNA- oder RNA-Oligonucleotiden,
- Vitaminen, Provitaminen und Vitaminvorstufen der Gruppen B, E, H und K und den Estern der vorgenannten Substanzen,
- a-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- anorganischen und organischen UV-Filtersubstanzen,
- selbstbräunenden,
- hautberuhigenden und
- sebumregulierenden Wirkstoffen sowie
- Mischungen dieser Wirkstoffe.

15. Zusammensetzung gemäß einem der Ansprüche 11 - 14, **dadurch gekennzeichnet, dass** mindestens ein nichtionischer Emulgator mit einem HLB-Wert von 8 und darunter der allgemeinen Formel R¹ - O - R², in der R¹ eine primäre lineare Alkyl-, Alkenyl- oder Acylgruppe mit 20 - 30 C-Atomen, bevorzugt einen Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt, bevorzugt Behenylalkohol, enthalten ist.

16. Zusammensetzung gemäß einem der Ansprüche 11 - 15, **dadurch gekennzeichnet, dass** sie in Form einer lamellare Strukturen aufweisenden Öl-in-Wasser-Emulsion oder in Form einer Wasser-in-Öl-Emulsion vorliegt.
